(19) Europäisches Patentamt
European Patent Office
Office européen des brevets

(11) EP 3 300 794 A2

(12) EUROPEAN PATENT APPLICATION

(43) Date of publication:
**04.04.2018 Bulletin 2018/14**

(21) Application number: **17193600.8**

(22) Date of filing: **27.09.2017**

(51) Int Cl.:
***B01J 13/14*** (2006.01) ***B01J 13/18*** (2006.01)
***A01N 25/28*** (2006.01) ***A61K 8/11*** (2006.01)
***A61K 9/50*** (2006.01) ***C11D 3/50*** (2006.01)
***D06M 23/12*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.09.2016 US 201615278805**

(71) Applicant: **International Flavors & Fragrances Inc.**
**New York, NY 10019 (US)**

(72) Inventors:
- **SASAKI, Takashi**
**Matawan, NJ New Jersey 07747 (US)**
- **LIN, Yuchuan**
**East Brunswick, NJ New Jersey 08816 (US)**
- **PLUYTER, Johan G. L.**
**Lindenhurst, IL Illinois 60046 (US)**

(74) Representative: **Barker Brettell LLP**
**100 Hagley Road**
**Edgbaston**
**Birmingham B16 8QQ (GB)**

# (54) MICROCAPSULE COMPOSITIONS CONTAINING AMINO SILICONE

(57) Disclosed is a microcapsule composition containing a microcapsule and an aminopolysiloxane. The microcapsule has an oil core including an active material and a capsule wall encapsulating the oil core. The microcapsule wall is formed of an encapsulating polymer. Also disclosed are a method of preparing the microcapsule composition and a consumer product containing the microcapsule composition.

EP 3 300 794 A2

## Description

## BACKGROUND

Nano- or micro-encapsulation is used in a variety of different applications where there is a need to deliver, apply, or release a fragrance or other active material at all stages of use in a time-delayed or controlled manner.

In a laundry application, it is desirable that a consumer can enjoy a pleasing scent from damp, freshly dried, and also post-storage fabrics. Current microcapsules do not release a fragrance during the use cycle of fabrics, spanning washing, drying, storing, and wearing.

Polyurea and polyurethane microcapsules have been developed to provide good performance on dry fabrics but not damp fabrics. See WO 2011/154893, WO 2012/107323, US 2011/0077188, US 5,635,211, US 6,586,107, and US 6,797,670. On the other hand, silica gel microcapsules impart a fresh scent to damp fabrics but not dry fabrics. See US 2014/0044760 and US 9,044,732. Simple mixing the polyurea/polyurethane and silica gel microcapsules cannot achieve a desirable performance.

There is a need to develop a capsule that provides lasting releasing of a fragrance at damp and dry stages.

## SUMMARY OF THE INVENTION

This invention is based on the discovery that certain capsule compositions deliver fragrance at both the damp and dry stages with high performance.

Accordingly, one aspect of this invention relates to a microcapsule composition comprising a microcapsule and an aminopolysiloxane. The microcapsule, having a particle size of 0.1 to 1000 microns, includes an oil core containing an active material and a capsule wall encapsulating the oil core. The microcapsule wall is formed of an encapsulating polymer.

The active material is typically a fragrance, pro-fragrance, flavor, malodor counteractive agent, vitamin or derivative thereof, anti-inflammatory agent, fungicide, anesthetic, analgesic, antimicrobial active, anti-viral agent, anti-infectious agent, anti-acne agent, skin lightening agent, insect repellant, animal repellent, vermin repellent, emollient, skin moisturizing agent, wrinkle control agent, UV protection agent, fabric softener active, hard surface cleaning active, skin or hair conditioning agent, flame retardant, antistatic agent, nanometer to micron size inorganic solid, polymeric or elastomeric particle, taste modulator, cell, probiotic, or combination thereof.

Examples of the encapsulating polymer include, but are not limited to, a polyacrylate, polyurea, polyurethane, polyacrylamide, polyester, polyether, polyamide, poly(acrylate-co-acrylamide), starch, silica, gelatin and gum Arabic, alginate, chitosan, polylactide, poly(melamine-formaldehyde), poly(urea-formaldehyde), and combinations thereof.

In some embodiments, the encapsulating polymer is a polyurea or polyurethane. The polyurea can be a reaction product of a polyfunctional isocyanate and a polyfunctional amine in the presence of an alkylnaphthalenesulfonate formaldehyde condensate and polyvinylpyrrolidone. The polyurethane can be a reaction product of a polyfunctional isocyanate and a polyfunctional alcohol as a cross-linking agent in the presence of an alkylnaphthalenesulfonate formaldehyde condensate and polyvinylpyrrolidone.

The polyfunctional isocyanate includes aromatic polyfunctional isocyanates, aliphatic polyfunctional isocyanates, and combinations thereof. The aromatic polyfunctional isocyanate typically contains a phenyl, tolyl, xylyl, naphthyl, or diphenyl moiety, or a combination. Non-limiting examples are polymeric methylene diphenyl diisocyanate, polyisocyanurates of toluene diisocyanate, trimethylol propane-adducts of toluene diisocyanate, trimethylol propane-adducts of xylylene diisocyanate, and combinations thereof. Examples of the aliphatic polyfunctional isocyanate are trimers of hexamethylene diisocyanate, trimers of isophorone diisocyanate, biurets of hexamethylene diisocyanate, and combinations thereof, especially dimers, biurets, symmetric trimers, and asymmetric trimers of hexamethylene diisocyanate

Suitable polyfunctional amines by way of illustration are hexamethylenediamine, hexaethylenediamine, ethylenediamine, 1,3-diaminopropane, 1,4-diamino-butane, diethylenetriamine, pentaethylenehexamine, 1,6-diaminohexane, hydrazine, 1,4-diaminocyclohexane and 1,3-diamino-1-methylpropane, bis(3-aminopropyl)amine, bis(hexamethylene)triamine, tris(2-aminoethyl)amine, triethylene-tetramine, N,N'-bis(3-aminopropyl)-1,3-propanediamine, tetraethylenepentamine, branched polyethylenimine, chitosan, nisin, gelatin, 1,3-diamino-guanidine, 1,1-dimethylbiguanide, guanidine, arginine, lysine, ornithine, histidine, amino-2-methyl-1-propanol, or a combination thereof.

When the alkylnaphthalenesulfonate formaldehyde condensate and polyvinylpyrrolidone are used as the dispersant, they are independently present at 0.1 to 5% by weight of the microcapsule composition. The ratio between the alkylnaphthalenesulfonate formaldehyde condensate and polyvinylpyrrolidone is 10 : 1 to 1 : 10.

In one embodiment, the encapsulating polymer is a polyurea that is a reaction product of a polyfunctional isocyanate and a polyfunctional amine, in which the polyfunctional isocyanate contains a trimethylol propane-adduct of toluene diisocyanate or a trimethylol propane-adduct of xylylene diisocyanate, and the polyfunctional amine is diethylenetriamine, bis(3-aminopropyl)amine, bis(hexamethylene)triamine, tris(2-aminoethyl)amine, triethylenetetramine,

N,N'-bis(3-aminopropyl)-1,3-propanediamine, tetraethylenepentamine, pentaethylenehexamine, branched polyethylenimine, chitosan, nisin, gelatin, 1,3-diaminoguanidine monohydrochloride, 1,1-dimethylbiguanide hydrochloride, or guanidine carbonate, or mixture thereof.

In another embodiment, the encapsulating polymer contains a first polymer and a second polymer, the ratio between the first polymer and the second polymer is 1 : 10 to 10 : 1, the first polymer is a sol-gel polymer such as a silica gel or polyalkylsiloxane, and the second polymer is polyacrylate, polyacrylamide, poly(acrylate-co-acrylamide), polyurea, polyurethane, starch, gelatin and gum Arabic, poly(melamine-formaldehyde), poly(urea-formaldehyde), or a combination thereof.

Optionally, the microcapsule composition contains a polyvinyl alcohol, polystyrene sulfonate, carboxymethyl cellulose, sodium polystyrene sulfonate, alkylnaphthalenesulfonate formaldehyde condensate, polyvinylpyrrolidone, copolymer of vinyl pyrrolidone and quaternized dimethylaminoethyl methacrylate, or combination thereof.

The microcapsule composition can further contain a cationically modified polyvinyl alcohol or quaternized polysaccharide.

Furthermore, the microcapsule composition can contain a deposition aid that is polyquaternium-4, polyquaternium-5, polyquaternium-6, polyquaternium-7, polyquaternium-10, polyquaternium-16, polyquaternium-22, polyquaternium-24, polyquaternium-28, polyquaternium-39, polyquaternium-44, polyquaternium-46, polyquaternium-47, polyquaternium-53, polyquaternium-55, polyquaternium-67, polyquaternium-68, polyquaternium-69, polyquaternium-73, polyquaternium-74, polyquaternium-77, polyquaternium-78, polyquaternium-79, polyquaternium-80, polyquaternium-81, polyquaternium-82, polyquaternium-86, polyquaternium-88, polyquaternium-101, polyvinylamine, polyethyleneimine, polyvinylamine and vinylformamide copolymer, an acrylamidopropyltrimonium chloride/acrylamide copolymer, a methacrylamidopropyltrimonium chloride/acrylamide copolymer, or a mixture thereof.

Turning to the aminopolysiloxane, one example is the polymer represented by the structure of Formula I:

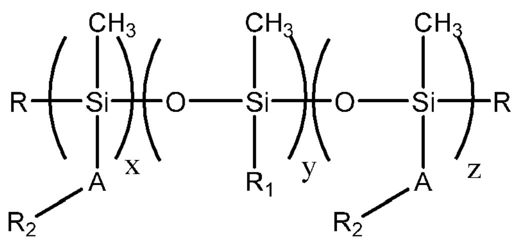

Formula I,

wherein R is $C_1$-$C_6$ alkyl, $C_1$-$C_6$ alkoxy, or -O-Si($CH_3$)$_3$; $R_1$ is $C_1$-$C_6$ alkyl, $C_1$-$C_6$ alkoxy, -$(OCH_2CH_2)_mH$, or -$(OCH_2CH_2CH_2)_n$-H, in which each of m and n, independently, is an integer from 0 to 10; $R_2$ is NH2, NHR', or NR'R", in which each of R' and R', independently, is a $C_1$-$C_6$ alkyl; A is $C_1$-$C_6$ alkyl, $C_1$-$C_6$ alkoxy, -$(OCH_2CH_2)_m$-H, or -$(OCH_2CH_2CH_2)_n$-H; x is 0 to 2000; y is 1-2000; and z is 1-2000.

Another aspect of this invention relates to a method of preparing the microcapsule composition described above. The method includes the steps of (a) providing an oil phase having an active material, a first polymer precursor, and a second polymer precursor, (b) providing an aqueous phase having an aminopolysiloxane (*e.g.*, at a level of 0.1 to 10% by weight of the microcapsule composition) and optionally, a cationically modified polyvinyl alcohol or quaternized polysaccharide, (c) emulsifying the oil phase into the aqueous phase to form an oil-in-water emulsion, (d) causing the formation of a capsule having an oil core that contains the active material and a capsule wall that is formed of the first polymer precursor and a second polymer precursor, and (e) curing the capsule to obtain the microcapsule composition, *e.g.*, at 40 to 250 °C for 10 minutes to 5 hours.

Another method of preparing the microcapsule composition includes the steps of (a) providing an oil phase having an active material and a second polymer precursor, (b) providing an aqueous phase having an aminopolysiloxane, (c) emulsifying the oil phase into the aqueous phase to form an oil-in-water emulsion, (d) adding a first polymer precursor to the oil-in-water emulsion, (e) causing the formation of a capsule having an oil core that contains the active material and a capsule wall that is formed of the first polymer precursor and a second polymer precursor, and (f) curing the capsule to obtain the microcapsule composition.

Alternatively, the first polymer precursor is added to the aqueous phase instead of the oil phase or the oil-in-water emulsion.

The first polymer precursor is a sol-gel precursor. The second polymer precursor is an acrylate monomer, acrylamide monomer, polyfunctional isocyanate, starch, gelatin-gum arabic, melamine-formaldehyde precondensate, urea-formaldehyde precondensate, or a combination thereof.

The method of this invention can further comprising (i) the step of (c-1) adding an activation agent to the oil-in-water emulsion before step (d), (ii) the step of (e-2): spray drying the capsule slurry after step (e), and/or (iii) (g) adding a rheology modifier to the microcapsule composition. The rheology modifier can be added before, after, or during any of the steps (a)-(f). Examples of the rheology modifier are alkali-swellable anionic acrylic polymer emulsion, anionic hydrophobically modified alkali-soluble acrylic polymer emulsion, anionic acrylic copolymer emulsion, hydrophobically-modified ethoxylated urethane, xanthan gum, carrageenan, gellan, pectin, hydroxyethyl cellulose, sodium carboxymethyl cellulose, guar, sodium alginate, fully exfoliated smectite clays, and combinations thereof.

Also within the scope of this invention is a consumer product containing any microcapsule composition described above. The consumer product can be a shampoo, hair conditioner, bar soap, liquid detergent, powder detergent, fabric conditioner, or fabric refresher.

The details of one or more embodiments of the invention are set forth in the description below. Other features, objects, and advantages of the invention will be apparent from the description and the claims.

## DETAILED DESCRIPTION OF THE INVENTION

It has been found that certain microcapsule compositions are suitable for delivering various hydrophobic or hydrophilic active materials for use in consumer products especially fabric care products.

Microcapsule compositions of this invention are useful in a wide range of consumer applications, e.g., personal care products including shampoos, hair conditioners, hair rinses, hair refreshers; personal wash such as bar soaps, body wash, personal cleaners and sanitizers, hydroalcoholic formulations; fabric care such as fabric refreshers, softeners and dryer sheets, ironing water, industrial cleaners, liquid and powder detergent including unit dose capsules, rinse conditioners, and scent booster products; fine fragrances; an Eau De Toilette products; deodorants; roll-on products, and aerosol products.

The microcapsule compositions each contain a microcapsule preferably having a size in the range of from 0.01 to 1000 microns in diameter (e.g., 0.5 to 1000 microns, 1 to 200 microns, 0.5 to 150 microns, 0.1 to 100 microns, 2 to 50 microns, 5 to 25 microns, 2 to 15 microns, and 1 to 10 microns). The microcapsule size distribution can be narrow, broad, or multi-modal.

The microcapsule includes an oil core and a capsule wall encapsulating the oil core.

The oil core contains an active material selected from the group consisting of a fragrance, pro-fragrance, flavor, malodor counteractive agent, UV absorber, anti-inflammatory agent, anesthetic, analgesic, biocide, anti-viral agent, anti-bacterial agent, anti-infectious agent, anti-acne agent, skin lightening agent, insect repellant, insecticides, emollient, skin moisturizing agent, detergent, silicone conditioner, shampoo, vitamin or derivative thereof, fat, oil, nutrient, enzyme, phase change material, dye, adhesive, corrosion inhibitor, anti-fouling agent, cosmetic active, oxidizing agent, personal care active, medicine, agrochemical, fertilizer, liquid crystal, printing ink, paint, rustproofing agent, recording material, catalyst, chemical reactant, magnetic substance, nanometer to micron size inorganic solid, polymeric or elastomeric particle, and any combinations thereof.

The active material is present at a level of 5 to 95% (preferably 20 to 90% and more preferably 40 to 85%) by weight of the capsule.

As to the capsule wall, it is formed of an encapsulating polymer selected from the group consisting of a polyacrylate, polyurea, polyurethane, polyacrylamide, polyester, polyether, polyamide, poly(acrylate-co-acrylamide), starch, silica, gelatin and gum Arabic, alginate, chitosan, polylactide, poly(melamine-formaldehyde), poly(urea-formaldehyde), and combinations thereof.

In some embodiments, the encapsulating polymer is a polyurea polymer that is a reaction product between a polyfunctional isocyanate and a polyfunctional amine in the presence of a dispersant.

The polyfunctional isocyanate includes an aromatic polyfunctional isocyanate, aliphatic polyfunctional isocyanate, or combination thereof. The aromatic polyfunctional isocyanate contains a phenyl, tolyl, xylyl, naphthyl, or diphenyl moiety, or a combination. Examples include the aromatic polyfunctional isocyanate is selected from the group consisting of polymeric methylene diphenyl diisocyanate, polyisocyanurates of toluene diisocyanate, trimethylol propane-adducts of toluene diisocyanate, trimethylol propane-adducts of xylylene diisocyanate, and combinations thereof. The aliphatic polyfunctional isocyanate can be a trimer of hexamethylene diisocyanate, a trimer of isophorone diisocyanate, a biuret of hexamethylene diisocyanate, or a combination thereof including dimers, biurets, symmetric trimers, asymmetric trimers of hexamethylene diisocyanate.

Examples of the polyfunctional amine include hexamethylenediamine, hexaethylenediamine, ethylenediamine, 1,3-diaminopropane, 1,4-diamino-butane, diethylenetriamine, pentaethylenehexamine, 1,6-diaminohexane, hydrazine, 1,4-diaminocyclohexane and 1,3-diamino-1-methylpropane, bis(3-aminopropyl)amine, bis(hexamethylene)triamine, tris(2-aminoethyl)amine, triethylene-tetramine, N,N'-bis(3-aminopropyl)-1,3-propanediamine, tetraethylenepentamine, branched polyethylenimine, chitosan, nisin, gelatin, 1,3-diamino-guanidine, 1,1-dimethylbiguanide, guanidine, arginine, lysine, ornithine, histidine, amino-2-methyl-1-propanol, and a combination thereof.

Exemplary dispersants are a polyvinyl alcohol, polystyrene sulfonate, carboxymethyl cellulose, sodium polystyrene sulfonate, alkylnaphthalenesulfonate formaldehyde condensate, polyvinylpyrrolidone, copolymer of vinyl pyrrolidone and quaternized dimethylaminoethyl methacrylate, and combinations thereof. The dispersant (*e.g.*, alkylnaphthalenesulfonate formaldehyde condensate and polyvinylpyrrolidone) is typically present at 0.1 to 5% (*e.g.,* 0.2 to 4%, 0.5 to 4%, and 1 to 3%) by weight of the microcapsule composition. Preferably, a combination of the alkylnaphthalenesulfonate formaldehyde condensate and polyvinylpyrrolidone is used at the ratio of 10 : 1 to 1 : 10 (*e.g.,* 5 : 1 to 1 : 5 and 3 : 1 to 1 : 3).

In some embodiments, the microcapsule is a hybrid microcapsule, namely, the encapsulating polymer is formed of two or more different polymers, *e.g.*, a first polymer and a second polymer. The first polymer is a sol-gel polymer. Exemplary second polymers are polyacrylate, polyacrylamide, poly(acrylate-co-acrylamide), polyurea, polyurethane, starch, gelatin and gum Arabic, poly(melamine-formaldehyde), poly(urea-formaldehyde), and any combinations thereof.

The first polymer and the second polymer are both present in the capsule wall. They can be intertwined or cross-linked in the wall or form a layered structure. By way of illustration, the second polymer forms an inner layer of the capsule wall and the first capsule forms an outer layer of the capsule wall coating the inner wall. Alternatively, the first polymer forms an inner layer and the second polymer forms an outer layer. The layered structure is determined by various factors such as the dispersant used and its amount, the first or second polymer, the shear mixing rate, the temperature, the ratio between the oil phase and the water phase, and etc.

As another example: the first polymer forms a first polymer network; the second polymer forms a second polymer network. The first polymer network is connected to the second polymer network via covalent or non-covalent bonding. Both the first and second polymer networks appear as patches side-by-side on the surface of the capsule.

In some embodiments, the capsule wall has an inner layer formed of a sol-gel polymer and an outer layer formed of a polyurea polymer. In other embodiments, the capsule wall is single-layers formed of a sol-gel polymer cross-linked with a polyurea polymer as patches on the surface of the capsule wall.

The capsule wall can also include one or more additional wall polymers, *e.g.*, a third, fourth, fifth, or sixth polymer. These additional polymers can be selected from the group consisting of polyacrylate, polyacrylamide, poly(acrylate-co-acrylamide), polyurea, polyurethane, starch, gelatin and gum Arabic, poly(melamine-formaldehyde), poly(urea-formaldehyde), and any combinations thereof.

The microcapsule composition of this invention contains an amino silicone, *e.g.*, aminopolysiloxane. Suitable amino silicones include aminated polydimethylsiloxane sold by Siltech Corporation as Salamine T-SA, PEG-8 distearmonium chloride PG-dimethicone such as Silquat J208-1B (Siltech Corporation; CAS# 1034336-15-6 / 107-41-5), silicone quaternium-8 such as Silquat AD (Siltech Corporation; CAS# 280112-39-2 / 7732-18-5), and bis-aminopropyl dimethicone silamine 2972 (Siltech Coproation; CAS# 192888-42-9).

The aminopolysiloxane can be added after the microcapsule is formed. It stabilizes the microcapsule slurry partially due to its amino functional group, which becomes positively charged in the microcapsule composition of this invention when the microcapsule composition has a pH of 8 or under (*e.g.,* pH 7 or under, pH 6.5 or under, and pH 6 or under). The aminopolysiloxane remains as a standalone free polymer, *i.e.,* not bonding to the microcapsule wall via a covalent bond. It either forms a coating over the microcapsule via electrostatic interactions or is freely suspended in the microcapsule composition.

Alternatively, the aminopolysiloxane is added as a co-dispersant during the preparation of the microcapsule, more specifically as an emulsifier for preparing a stable oil-in-water emulsion. Typically, the aminopolysiloxane does not react with any of the wall materials or precursors such as polyfunctional isocyanates and polyfunctional amines. It remains as a standalone free polymer, possibly on the interface between the microcapsule and the aqueous phase that the microcapsule disperses in.

Oftentimes, when used as a co-dispersant, the aminopolysiloxane is added together with a cationic polymer such as a cationically modified vinyl amine/vinyl alcohol copolymer (SelvolTM Ultralux AD commercially available from Sekisui Specialty Chemicals America), polyquaternium-10 (a quaternized hydroxyethyl cellulose commercially available from Dow as UCARETM Polymer JR-30M).

The microcapsule composition typically contains the aminopolysiloxane at a level of 0.1 to 10% (*e.g.,* 0.1 to 5%, 0.2 to 4%, and 0.3 to 3%) by weight of the microcapsule composition.

The microcapsule composition can also contain the cationic polymer at a level of 0.1 to 10% (*e.g.,* 0.1 to 5%, 0.2 to 4%, and 0.3 to 3%).

The total amount of the aminopolysiloxane and cationic polymer, in some embodiments, constitutes 0.2 to 10% (*e.g.,* 0.3 to 6%, 0.5 to 5%, and 0.8 to 4%).

The capsule composition optionally contains a rheology modifier to the microcapsule composition, wherein the rheology modifier is selected from the group consisting of alkali-swellable anionic acrylic polymer emulsion, anionic hydrophobically modified alkali-soluble acrylic polymer emulsion, anionic acrylic copolymer emulsion, hydrophobically-modified ethoxylated urethane, xanthan gum, carrageenan, gellan, pectin, hydroxyethyl cellulose, sodium carboxymethyl cellulose, guar, sodium alginate, fully exfoliated smectite clays, and combinations thereof.

Conventional encapsulation methods can be used to prepare the capsule microcapsule composition of this invention. See US 2014/0287008, 2014/0044761, and 2011/0033513. In some embodiments, capsule formation aids,

*e.g.*, a surfactant or dispersant, are used.

Hybrid microcapsules can be prepared by reacting a sol-gel precursor (*i.e.,* a first wall-forming material) and a second polymer precursor (*i.e.,* a second wall-forming material) in the presence or absence of an activation agent. By way of illustration, to prepare a hybrid capsule, an oil phase is first provided that has an active material, a sol-gel precursor as a first polymer precursor, and a polyisocyanate as a second polymer precursor. A water phase containing an emulsifier is then blended with the oil phase and emulsified to form an oil-in-water emulsion. A polyfunctional amine is added to the emulsion as a crosslinking agent to cause the formation of polyurea by crosslinking the polyisocyanate. A sol-gel polymer is also formed by the reaction between the sol-gel precursor and water, which already exists in the emulsion or, optionally, freshly added to the emulsion. Crosslinking between the sol-gel precursor and the polyisocyanate can also take place in the presence or absence of a catalyst. The resultant capsule slurry is then cured at a predetermined temperature for a predetermined period of time. In accordance with some embodiments of this invention, the capsules can be cured at a temperature in the range of, *e.g*., 15°C to 130°C (e.g., 55°C to 90°C, 55°C to 75°C, and 90°C to 130°C) for 1 minute to 10 hours (e.g., 0.1 hours to 5 hours, 0.2 hours to 4 hours and 0.5 hours to 3 hours). A skilled person in the art can determine, without undue experimentation, the curing temperature, duration, and the heating rate.

To obtain capsules with more leaching of the active material, certain embodiments of this invention provide for a cure temperature of 100°C or less. In some embodiments, the cure temperature is 90°C or less. In other embodiments, the cure temperature is 80°C or less.

In one embodiment, the capsules are heated to a target cure temperature at a linear rate of 0.5 to 2 °C per minute (e.g., 1 to 5 °C per minute, 2 to 8 °C per minute, and 2 to 10°C per minute) over a period of 1 to 60 minutes (e.g., 1 to 30 minutes). The following heating methods may be used: conduction for example via oil, steam radiation via infrared, and microwave, convection via heated air, steam injection and other methods known by those skilled in the art. The target cure temperature used herein refers to the minimum temperature in degrees Celsius at which the capsules may be cured to retard leaching.

In some embodiment, the microcapsule composition contains two or more microcapsules, *e.g*., a first microcapsule, a second microcapsule, a third microcapsule, and a fourth microcapsule. These microcapsules have a release profile different from each other. In one embodiment, the first microcapsule is cured at a temperature of 100 °C or greater (*e.g.,* 105 °C or greater, 110 °C o greater, 120 °C or greater, 130 °C or greater, and 105 to 140 °C.). The second microcapsule is cured at a temperature of 100 °C or less (*e.g.,* 90 °C or less, 80 °C or less, 70 °C or less, 60 °C or greater, and 50 to 95 °C). The first and second microcapsules each have a microcapsule wall formed of the same or different encapsulating polymers such as melamine-formaldehyde polymers, silica, polyurea, and polyacrylate as those described in US Patent Application Publication Nos. 20070138673, 20110033513, 20140044760, 20150252312, 20160193122, and 20150203787. The contents of these publications are herein incorporated by reference in their entirety. In one embodiment, the first and second microcapsules each are formed of the same polymer, e.g., a melamine-formaldehyde or polyurea polymer and cured at a different temperature. In another embodiment, the first microcapsule has a microcapsule wall formed of a melamine-formaldehyde polymer and the second microcapsule has a microcapsule wall formed of a polyurea.

The microcapsule composition of this invention optionally contains other additional viscosity control agent or stabilizing agent. Suitable viscosity control agents include an acrylate copolymer, a cationic acrylamide copolymer, a polysaccharide, or a combination thereof.

Commercially available acrylate copolymers include those under the trade name ACULYNTM (from Dow Chemical Company) such as ACULYNTM 22 (a copolymer of acrylates and stearth-20 methacrylate), ACULYNTM 28 (a copolymer of acrylate and beheneth-25 methacrylate), ACULYNTM 33 (a copolymer of acrylic acid and acrylate), ACULYNTM 38 (a crosspolymer of acrylate and vinyl neodecanoate), and ACULYNTM 88 (a crosspolymer of acrylate and steareth-20 methacrylate). Particularly useful acrylate copolymers are anionic acrylate copolymer such as ACULYNTM 33, an alkali-soluble anionic acrylic polymer emulsion (ASE), which is synthesized from acrylic acid and acrylate comonomers through emulsion polymerization. The commercial product of ACULYNTM 33 contains 28% of the polymer in water, has a pH of 3, a density of 1.05 g/mL, an equivalent weight of 218 (the equivalent weight refers to grams of dry polymer neutralized by 1 equivalent, i.e., 40 grams of sodium hydroxide), and a viscosity of 10 cP. In one embodiment, the acrylate copolymer has the following structure:

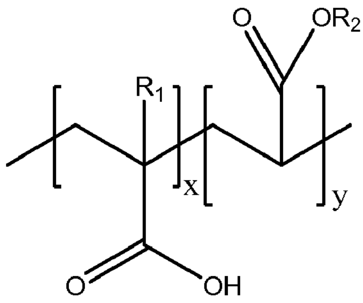

,

in which, R1 is H or a C1-C24 (e.g., C1-C10 and C1-C6) alkyl, R2 is a C1-C24 (e.g., C1-C10 and C1-C6) alkyl, x is an integer selected from 1-4100, and y is an integer selected from 1-4100. The molecular weight of the acrylate copolymer is 200-10000000 (e.g., 500-5000000, 1000-1000000, and 2000 to 500000).

Carbopol® polymers are also suitable acrylate copolymer useful in this invention. Examples are Carbopol® ETD 2020 polymer (a crosspolymer of acrylate and C10-C30 alkyl acrylate), Carbopol® ETD 2691, Carbopol® ETD 2623 (a crosslinked acrylate copolymer),

Carbomer (polyacrylic acid) can also be used as viscosity control agents. Carbomer codes (672, 690, 910, 934, 934P, 940, 941, 1342, 1662) are an indication of molecular weight and the specific components of the polymer. Their molecular weight ranges from 100000 to 3,000,000 (Carbomer 672, M.W. 3,000,000; Carbomer 910, M.W., 750,000; Carbomer 934, M.W., 500000; Carbomer 940, M.W. 4000000; Carbomer 941, M.W. 1250000; and Carbomer 1662, M.W., 4,000,000). Carbomer polymers are commercially available, e.g., under the trade name Carbopol® from Lubrizol Corporation.

Polysaccharides are another class of agents suitable for viscosity control agents. Commonly used polysaccharides include starches, pectin, and vegetable gums such as alginin, guar gum, locust bean gum, and xanthan gum (e.g., Keltrol® T, 80-mesh food-grade, commercially available from CP Kelco, Atlanta, Georgia).

Cationic acrylamide copolymers can also be used as viscosity control agents. These cationic cross-linked polymers are derivable from the polymerization of from 5 to 100 mole percent of cationic vinyl addition monomer, from 0 to 95 mole percent of acrylamide and from 50 to 1000 ppm of a difunctional vinyl addition monomer cross-linking agent. Preferred polymers are cross-linked copolymers of acrylamide and methacrylate cross-linked with a difunctional vinyl addition monomer, such as methylene bisacrylamide. Particularly preferred polymers are copolymers of 20 % acrylamide and 80 % MADAM methyl chloride (MADAM: dimethyl amino ethyl methacrylate) cross-linked with from 450 to 600 ppm of methylene bisacrylamide. In one embodiment, the cationic acrylamide copolymer is a cationic copolymer obtained by Hofmann rearrangement in aqueous solution in the presence of an alkali and/or alkaline earth hydroxide and an alkali and/or alkaline earth hypohalide, on a base copolymer comprising: (i) at least 5 mole % of a non-ionic monomer selected from the group consisting of acrylamide, methacrylamide, N,N-dimethylacrylamide, acrylonitrile, and combinations thereof; and (ii) at least one comonomer selected from the group consisting of unsaturated cationic ethylenic comonomer, non-ionic comonomer, or combinations thereof, provided that the non-ionic comonomer is not acrylamide, methacrylamide, N,N-dimethylacrylamide, or acrylonitrile. The cationic copolymer thus obtained has a desalination coefficient (Cd) of greater than 0.6 (e.g., greater than 0.65 and greater than 0.7). Cd is calculated as Real polymeric active matter (% by weight of the copolymer) $\times$ Polymer filler density Conductivity of the solution containing 9% of active matter. See also US 8,242,215. The unsaturated cationic ethylenic comonomer can be selected from the group consisting of dialkylaminoalkyl(meth)acrylamide monomers, diallylamine monomers, methyldiallylamine monomers, and quaternary ammonium salts or acids thereof, such as dimethyldiallylammonium chloride (DADMAC), acrylamidopropyltrimethylammonium chloride (APTAC), methacrylamidopropyltrimethylammonium chloride (MAPTAC). Examples of the non-ionic comonomer are N-vinyl acetamide, N-vinyl formamide, N-vinylpyrrolidone, vinyl acetate, and combinations thereof. The base copolymer is preferably branched in the presence of a branching agent selected from the group consisting of methylene bisacrylamide, ethylene glycol di-acrylate, polyethylene glycol dimethacrylate, diacrylamide, cyanomethylacrylate, vinyloxyethylacrylate, vinyloxyethylmethacrylate, triallylamine, formaldehyde, glyoxal, and a glycidylether type compound. More examples of the cationic acrylamide copolymers can be found in US 8,242,215. Such materials are commercially available from SNF Floerger under the trade names Flosoft FS 200, Flosoft FS 222, and Flosoft FS 228. See also WO 2007141310, US 20060252668, and US 20100326614.

Additional examples of the viscosity control agent include polypropylene glycol, materials containing propylene oxide groups, materials containing polyethylene oxide groups, polysorbate 20 (TWEEN™ 20), POLOXAMER™ 124 (PLURONIC™ L44) polyethylene oxide-polypropylene oxide block copolymer having the formula (EO)x(PO)y(EO)z with x=11$\pm$3, z=11$\pm$3 and y=21$\pm$5, POLOXAMER™ L35, POLOXAMER™ L31, polyethylene glycol 55 (PEG-55), glycerin, diethylene glycol, CREMOPHOR™ polyoxyethyleneglyceroltriricinoleat, GLUCAM™ P-10 propylene glycol ether of methyl glucose with 10 polypropylene oxide units, PLURIOL™ E300 alkoxylates based on ethylene oxide and propylene

oxide, sodium cumene sulfonate (SCS), sodium xylene sulfonate (SXS), GLUCAM™ P-20 propylene glycol ether of methyl glucose with 20 polypropylene oxide units, GLUCAM™ E-20 ethylene glycol ether of methyl glucose with 20 polyethylene oxide units, GLUCAM™ E-10 ethylene glycol ether of methyl glucose with 10 polyethylene oxide units, and short chain ethoxylated propoxylated alcohols such as PPG2-Buteth-3, PPG3-Buteth-5, or PPG5-Buteth-7. More viscosity control agents are described in US 6,465,416 and US 20060252668.

Clays can also be used as a viscosity control agent. Commercially available montmorillonite clays include GELWHITE series (highly purified montmorillonite clays) marketed as GELWHITE-GP, GELWHITE-H, and GELWHITE-L by BYK Additives & Instruments, Germany. Other commercial products include Mineral Colloid BP, Mineral Colloid MO, Gelwhite MAS 100 (sc), Gelwhite MAS 101, Gelwhite MAS 102, Gelwhite MAS 103, Bentolite WH, Bentolite L10, Bentolite H, Bentolite L, Permont SX10A, Permont SC20, and Permont HN24 (Southern Clay Products, Texas, USA); Bentone EW and Bentone MA (Dow Corning); Bentonite USP BL 670 and Bentolite H4430 (Whitaker, Clarke & Daniels); Clarit 100 G1 and Clarit 1100 G1 (calcium bentonites from Süd Chemie AG); and Volclay 2 (sodium bentonite from Süd Chemie AG).

Materials for preparing the hybrid capsules are described below in details.

*Sol-gel Precursors*

Suitable sol-gel precursors are compounds capable of forming gels such as compounds containing silicon, boron, aluminum, titanium, zinc, zirconium, and vanadium. Preferred precursors are organosilicon, organoboron, and organoaluminum including metal alkoxides and b-diketonates.

Sol-gel precursors suitable for the purposes of the invention are selected in particular from the group of di-, tri- and/or tetrafunctional silicic acid, boric acid and alumoesters, more particularly alkoxysilanes (alkyl orthosilicates), and precursors thereof.

One example of sol-gel precursors suitable for the purposes of the invention are alkoxysilanes corresponding to the following general formula:

$$(R_1O)(R_2O)M(X)(X'),$$

wherein X can be hydrogen or $-OR_3$; X' can be hydrogen or $-OR_4$; and $R_1$, $R_2$, $R_3$ and $R_4$ independently represent an organic group, more particularly a linear or branched alkyl group, preferably a $C_1$-$C_{12}$ alkyl. M can be Si, Ti, or Zr.

A preferred sol/gel precursor is alkoxysilanes corresponding to the following general formula: $(R_1O)(R_2O)Si(X)(X')$, wherein each of X, X', $R_1$, and $R_2$ are defined above.

Particularly preferred compounds are the silicic acid esters such as tetramethyl orthosilicate (TMOS) and tetraethyl orthosilicate (TEOS). A preferred compound includes Dynasylan® (organofunctional silanes commercially available from Degussa Corporation , Parsippany New Jersey, USA). Other sol-gel precursors suitable for the purposes of the invention are described, for example, in German Patent Application DE10021165. These sol-gel precursors are various hydrolyzable organosilanes such as, for example, alkylsilanes, alkoxysilanes, alkyl alkoxysilanes and organoalkoxysilanes. Besides the alkyl and alkoxy groups, other organic groups (for example allyl groups, aminoalkyl groups, hydroxyalkyl groups, etc.) may be attached as substituents to the silicon.

Recognizing that metal and semi metal alkoxide monomers (and their partially hydrolyzed and condensed polymers) such as tetramethoxy silane (TMOS), tetraethoxy silane (TEOS), etc. are very good solvents for numerous molecules and active ingredients is highly advantageous since it facilitates dissolving the active materials at a high concentration and thus a high loading in the final capsules.

*Polyacrylate/polyacrylamide/poly(acrylate-co-acrylamide) precursors*

Preferred polyacrylate precursor are bi- or polyfunctional vinyl monomers including by way of illustration and not limitation, allyl methacrylate/acrylamide, triethylene glycol dimethacrylate/acrylamide, ethylene glycol dimethacrylate/acrylamide, diethylene glycol dimethacrylate/acrylamide, triethylene glycol dimethacrylate/acrylamide, tetraethylene glycol dimethacrylate/acrylamide, propylene glycol dimethacrylate/acrylamide, glycerol dimethacrylate/acrylamide, neopentyl glycol dimethacrylate/acrylamide, 1,10-decanediol dimethacrylate/acrylamide, pentaerythritol trimethacrylate/acrylamide, pentaerythritol tetramethacrylate/acrylamide, dipentaerythritol hexamethacrylate/acrylamide, triallylformal trimethacrylate/acrylamide, trimethylol propane trimethacrylate/acrylamide, tributanediol dimethacrylate/acrylamide, aliphatic or aromatic urethane diacrylates/acrylamides, difunctional urethane acrylates/acrylamides, ethoxylated aliphatic difunctional urethane methacrylates/acrylamides, aliphatic or aromatic urethane dimethacrylates/acrylamides, epoxy acrylates/acrylamides, epoxymethacrylates/acrylamides, 1,3-butylene glycol diacrylate/acrylamide, 1,4-butanediol dimethacrylate/acrylamide, 1,4-butaneidiol diacrylate/acrylamide, diethylene glycol diacrylate/acrylamide, 1,6-hexanediol diacrylate/acrylamide, 1,6-hexanediol dimethacrylate/acrylamide, neopentyl glycol diacrylate/acrylamide, polyethylene glycol diacrylate/acrylamide, tetraethylene glycol diacrylate/acrylamide, triethylene glycol diacrylate/acrylamide, 1,3-butylene glycol dimethacrylate/acrylamide, tripropylene glycol diacrylate/acrylamide, ethoxylated bisphenol diacrylate/acrylamide, ethoxylated bisphenol dimethylacrylate/acrylamide, dipropylene glycol diacrylate/acrylamide, alkoxylated hexanediol diacrylate/acrylamide, alkoxylated cyclohexane dimethanol diacrylate/acrylamide, propoxylated neopentyl glycol diacrylate/acrylamide, trimethylolpropane triacrylate/acrylamide, pentaerythritol triacrylate/acrylamide, ethoxylated trimethylolpropane triacrylate/acrylamide, propoxylated trimethylolpropane triacrylate/ acrylamide, propoxylated glyceryl triacrylate/acrylamide, ditrimethyloipropane tetraacrylate/ acrylamide, dipentaerythritol pentaacrylate/acrylamide, ethoxylated pentaerythritol tetraacrylate/ acrylamide, PEG 200 dimethacrylate/acrylamide, PEG 400 dimethacrylate/acrylamide, PEG 600 dimethacrylate/acrylamide, 3-acryloyloxy glycol monoacrylate/acrylamide, triacryl formal, triallyl isocyanate, and triallyl isocyanurate.

The monomer is polymerized in the presence of an activation agent (*e.g.,* an initiator) at a raised temperature (*e.g.*, 30-90 °C) or under UV light. Exemplary initiators are 2,2'-azobis(isobutyronitrile) ("AIBN"), dicetyl peroxydicarbonate, di(4-tert-butylcyclohexyl) peroxydicarbonate, dioctanoyl peroxide, dibenzoyl peroxide, dilauroyl peroxide, didecanoyl peroxide, tert-butyl peracetate, tert-butyl perlaurate, tert-butyl perbenzoate, tert-butyl hydroperoxide, cumene hydroperoxide, cumene ethylperoxide, diisopropylhydroxy dicarboxylate, 2,2'-azobis(2,4- dimethyl valeronitrile), 1,1 '-azobis(cyclohexane-1-carbonitrile), dimethyl 2,2'-azobis(2-methylpropionate), 2,2'-azobis[2-methyl-N-(2-hydroxyethyl) propionamide, sodium persulfate, benzoyl peroxide, and combinations thereof.

Emulsifiers used in the formation of polyacrylate/polyacrylamide/poly(acrylate-co-acrylamide) capsule walls are typically anionic emulsifiers including by way of illustration and not limitation, water-soluble salts of alkyl sulfates, alkyl ether sulfates, alkyl isothionates, alkyl carboxylates, alkyl sulfosuccinates, alkyl succinamates, alkyl sulfate salts such as sodium dodecyl sulfate, alkyl sarcosinates, alkyl derivatives of protein hydrolyzates, acyl aspartates, alkyl or alkyl ether or alkylaryl ether phosphate esters, sodium dodecyl sulphate, phospholipids or lecithin, or soaps, sodium, potassium or ammonium stearate, oleate or palmitate, alkylarylsulfonic acid salts such as sodium dodecylbenzenesulfonate, sodium dialkylsulfosuccinates, dioctyl sulfosuccinate, sodium dilaurylsulfosuccinate, poly(styrene sulfonate) sodium salt, isobutylene-maleic anhydride copolymer, gum arabic, sodium alginate, carboxymethylcellulose, cellulose sulfate and pectin, poly(styrene sulfonate), isobutylene-maleic anhydride copolymer, gum arabic, carrageenan, sodium alginate, pectic acid, tragacanth gum, almond gum and agar; semi-synthetic polymers such as carboxymethyl cellulose, sulfated cellulose, sulfated methylcellulose, carboxymethyl starch, phosphated starch, lignin sulfonic acid; and synthetic polymers such as maleic anhydride copolymers (including hydrolyzates thereof), polyacrylic acid, polymethacrylic acid, acrylic acid butyl acrylate copolymer or crotonic acid homopolymers and copolymers, vinylbenzenesulfonic acid or 2-acrylamido-2-methylpropanesulfonic acid homopolymers and copolymers, and partial amide or partial ester of such polymers and copolymers, carboxymodified polyvinyl alcohol, sulfonic acid-modified polyvinyl alcohol and phosphoric acid-modified polyvinyl alcohol, phosphated or sulfated tristyrylphenol ethoxylates. The amount of anionic emulsifier is anywhere from about 0.1 to about 40 percent by weight of all constitutents, more preferably from 0.5 to about 10 percent, more preferably 0.5 to 5 percent by weigh.

Polymeric stabilizers are often added to the silica hybrid capsules containing polyacrylate, polyacrylamide, or poly(acrylate-co-acrylamide). Suitable stabilizers are cationic cellulose derivatives, quaternized gums, polyethylene imines, cationic polyacrylates, polyacrylamides, polyacrylates, gelatin, quaternized protein hydrolysates, quaternized amino silicones, hydroxyethyl cellulose, polyvinyl pyrrolidone, poly vinyl alcohol, styrene co-polymer with maleic anhydride or acrylic acid, and combinations thereof.

*Polyurea/polyurethane precursors*

Suitable polyurea or polyurethane polymers are prepared using one or more polyisocyanates and one or more crosslinking agents.

(i) Polyisocyanates. Each of polyisocyanates has two or more isocyanate groups, i.e., O=C=N-, wherein said polyisocyanate can be aromatic, aliphatic, linear, branched, or cyclic. In certain embodiments, the polyisocyanate contains, on average, 2 to 4 -N=C=O groups. In particular embodiments, the polyisocyanate contains at least three isocyanate functional groups. In certain embodiments, the polyisocyanate is water insoluble.

The polyisocyanate can be an aromatic or aliphatic polyisocyanate. Desirable aromatic polyisocyanates each have a phenyl, tolyl, xylyl, naphthyl or diphenyl moiety as the aromatic component. In certain embodiments, the aromatic polyisocyanate is a polymeric methylene diphenyl diisocyanate ("PMDI"), a polyisocyanurate of toluene diisocyanate, a trimethylol propane-adduct of toluene diisocyanate or a trimethylol propane-adduct of xylylene diisocyanate.

Suitable aliphatic polyisocyanates include trimers of hexamethylene diisocyanate, trimers of isophorone diisocyanate or biurets of hexamethylene diisocyanate. Additional examples include those commercially available, e.g., BAYHYDUR N304 and BAYHYDUR N305, which are aliphatic water-dispersible polyisocyanates based on hexamethylene diisocyanate; DESMODUR N3600, DESMODUR N3700, and DESMODUR N3900, which are low viscosity, polyfunctional aliphatic polyisocyanates based on hexamethylene diisocyanate; and DESMODUR 3600 and DESMODUR N100 which are aliphatic polyisocyanates based on hexamethylene diisocyanate, commercially available from Bayer Corporation, Pittsburgh, PA).

Specific examples of wall monomer polyisocyanates include 1,5-naphthylene diisocyanate, 4,4'-diphenylmethane diisocyanate (MDI), hydrogenated MDI (H12MDI), xylylene diisocyanate (XDI), tetramethylxylol diisocyanate (TMXDI), 4,4'-diphenyldimethylmethane diisocyanate, di- and tetraalkyldiphenylmethane diisocyanate, 4,4'-dibenzyl diisocyanate, 1,3-phenylene diisocyanate, 1,4-phenylene diisocyanate, the isomers of tolylene diisocyanate (TDI), optionally in a mixture, 1-methyl-2,4-diisocyanatocyclohexane, 1,6-diisocyanato-2,2,4-trimethylhexane, 1,6-diisocyanato-2,4,4-trimethylhexane, 1-isocyanatomethyl-3-isocyanato-1,5,5-trimethylcyclohexane, chlorinated and brominated diisocyanates, phosphorus-containing diisocyanates, 4,4'-diisocyanatophenylperfluoroethane, tetramethoxybutane 1,4-diisocyanate, butane 1,4-diisocyanate, hexane 1,6-diisocyanate (HDI), dicyclohexylmethane diisocyanate, cyclohexane 1,4-diisocyanate, ethylene diisocyanate, phthalic acid bisisocyanatoethyl ester, also polyisocyanates with reactive halogen atoms, such as 1-chloromethylphenyl 2,4-diisocyanate, 1-bromomethylphenyl 2,6-diisocyanate, 3,3-bischloromethyl ether 4,4'-diphenyldiisocyanate. Sulfur-containing polyisocyanates are obtained, for example, by reacting 2 mol of hexamethylene diisocyanate with 1 mol of thiodiglycol or dihydroxydihexyl sulfide. Further suitable diisocyanates are trimethylhexamethylene diisocyanate, 1,4-diisocyanatobutane, 1,2-diisocyanatododecane and dimer fatty acid diisocyanate.

Other suitable commercially-available polyisocyanates include LUPRANATE M20 (PMDI, commercially available from BASF containing isocyanate group "NCO" 31.5 wt%), where the average n is 0.7; PAPI 27 (PMDI commercially available from Dow Chemical having an average molecular weight of 340 and containing NCO 31.4 wt%) where the average n is 0.7; MONDUR MR (PMDI containing NCO at 31 wt% or greater, commercially available from Bayer) where the average n is 0.8; MONDUR MR Light (PMDI containing NCO 31.8 wt%, commercially available from Bayer) where the average n is 0.8; MONDUR 489 (PMDI commercially available from Bayer containing NCO 30-31.4 wt%) where the average n is 1.0; poly[(phenylisocyanate)-co-formaldehyde] (Aldrich Chemical, Milwaukee, WI), other isocyanate monomers such as DESMODUR N3200 (poly(hexamethylene diisocyanate) commercially available from Bayer), and TAKENATE D110-N (xylene diisocyanate adduct polymer commercially available from Mitsui Chemicals corporation, Rye Brook, NY, containing NCO 11.5 wt%), DESMODUR L75 (a polyisocyanate base on toluene diisocyanate commercially available from Bayer), and DESMODUR IL (another polyisocyanate based on toluene diisocyanate commercially available from Bayer).

In some embodiments, the polyisocyanate used in the preparation of the capsules of this invention is a single polyisocyanate. In other embodiments the polyisocyanate is a mixture of polyisocyanates. In some embodiments, the mixture of polyisocyanates includes an aliphatic polyisocyanate and an aromatic polyisocyanate. In particular embodiments, the mixture of polyisocyanates is a biuret of hexamethylene diisocyanate and a trimethylol propane-adduct of xylylene diisocyanate. In certain embodiments, the polyisocyanate is an aliphatic isocyanate or a mixture of aliphatic isocyanate, free of any aromatic isocyanate. In other words, in these embodiments, no aromatic isocyanate is used to prepare the polyurea/polyurethane polymers as capsule wall materials.

The average molecular weight of certain suitable polyisocyanates varies from 250 to 1000 Da and preferable from 275 to 500 Da. In general, the range of the polyisocyanate concentration varies from 0.1% to 10%, preferably from 0.1% to 8%, more preferably from 0.2 to 5%, and even more preferably from 1.5% to 3.5%, all based on the weight of the capsule.

More examples of suitable polyisocyanates can be found in WO 2004/054362; WO 2015/023961; EP 0 148149; EP 0 017 409 B1; U.S. Pat. No. 4,417,916, U.S. Pat. No. 4,124,526, U.S. Pat. No. 5,583,090, U.S. Pat. No. 6,566,306, U.S. Pat. No. 6,730,635, PCT 90/08468, PCT WO 92/13450, U.S. Pat. No. 4,681,806, U.S. Pat. No. 4,285,720 and U.S. Pat. No. 6,340,653.

(ii) Crosslinking agents. The crosslinking agents each contain multiple *(i.e.,* two or more) functional groups (*e.g.*, -NH-, $-NH_2$ and -OH) that can react with polyisocyanates to form polyureas or polyurethanes. Examples include polyfunctional amines containing two or more amine groups (*i.e.*, polyamines), polyfunctional alcohols containing two or more hydroxyl groups (*i.e.,* polyols), and hybrid crosslinking agents containing one or more amine groups and one or more hydroxyl groups.

Amine groups in the crosslinking agents include $-NH_2$ and -R*NH, R* being substituted and unsubstituted $C_1$-$C_{20}$ alkyl, $C_1$-$C_{20}$ heteroalkyl, $C_1$-$C_{20}$ cycloalkyl, 3- to 8-membered heterocycloalkyl, aryl, and heteroaryl.

Two classes of such polyamines include polyalkylene polyamines having the following structures:

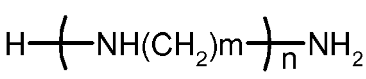

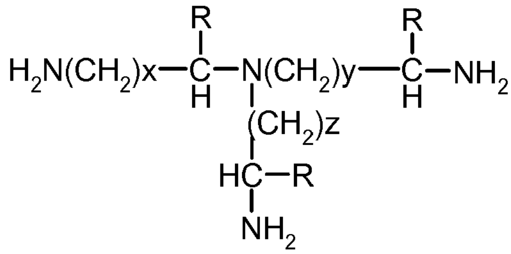

in which R is hydrogen or $-CH_3$; and m, n, x, y, and z each are integers from 0-2000 (e.g., 1, 2, 3, 4, and 5). Examples include ethylene diamine, 1,3-diaminepropane, diethylene triamine, triethylene tetramine, 1,4-diaminobutane, hexaethylene diamine, hexamethylene diamine, pentaethylenehexamine, and the like.

Another class of polyamines are polyalykylene polyamines of the type:

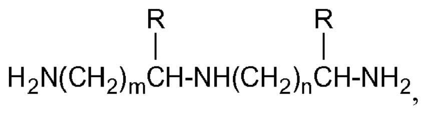

where R equals hydrogen or $-CH_3$, m is 1-5 and n is 1-5, *e.g.*, diethylene triamine, triethylene tetraamine and the like. Exemplary amines of this type also include diethylenetriamine, bis(3-aminopropyl)amine, bis(hexanethylene)triamine.

Another class of amine that can be used in the invention is polyetheramines. They contain primary amino groups attached to the end of a polyether backbone. The polyether backbone is normally based on either propylene oxide (PO), ethylene oxide (EO), or mixed PO/EO. The ether amine can be monoamine, diamine, or triamine, based on this core structure. An example is:

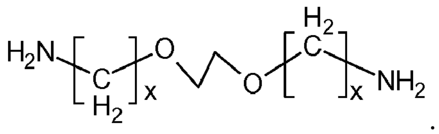

Exemplary polyetheramines include 2,2'-ethylenedioxy)bis (ethylamine) and 4,7,10-trioxa-1,13-tridecanediamine.

Other suitable amines include, but are not limited to, tris(2-aminoethyl)amine, triethylenetetramine, N,N'-bis(3-aminopropyl)-1,3-propanediamine, tetraethylene pentamine, 1,2-diaminopropane, N,N,N',N'-tetrakis(2-hydroxyethyl)ethylene diamine, N,N,N',N'-tetrakis(2-hydroxypropyl)ethylene diamine, branched polyethylenimine, 2,4-diamino-6-hydroxypyrimidine and 2,4,6-triaminopyrimidine.

Amphoteric amines, *i.e.*, amines that can react as an acid as well as a base, are another class of amines of use in this invention. Examples of amphoteric amines include proteins and amino acids such as gelatin, L-lysine, D-lysine, L-arginine, D-arginine, L-lysine monohydrochloride, D-lysine monohydrochloride, L-arginine monohydrochloride, D-arginine monohydrochloride, L-ornithine monohydrochloride, D-ornithine monohydrochloride or a mixture thereof.

Guanidine amines and guanidine salts are yet another class of multi-functional amines of use in this invention. Exemplary guanidine amines and guanidine salts include, but are not limited to, 1,3-diaminoguanidine monohydrochloride, 1,1-dimethylbiguanide hydrochloride, guanidine carbonate and guanidine hydrochloride.

Commercially available examples of amines include JEFFAMINE EDR-148 having a structure shown above (where x=2), JEFFAMINE EDR-176 (where x=3) (from Huntsman). Other polyether amines include the JEFFAMINE ED Series, JEFFAMINE TRIAMINES, polyethylenimines from BASF (Ludwigshafen, Germany) under LUPASOL grades (e.g., Lupasol FG, Lupasol G20 waterfree, Lupasol PR 8515, Lupasol WF, Lupasol FC, Lupasol G20, Lupasol G35, Lupasol G100, Lupasol G500, Lupasol HF, Lupasol PS, Lupasol HEO 1, Lupasol PN50, Lupasol PN60, Lupasol PO100 and Lupasol SK). Other commercially available polyethylenimines include EPOMIN P-1000, EPOMIN P-1050, EPOMIN RP18W and EPOMIN PP-061 from NIPPON SHOKUBAI (New York, NY). Polyvinylamines such as those sold by BASF under LUPAMINE grades can also be used. A wide range of polyetheramines may be selected by those skilled in the art. In certain embodiments, the cross-linking agent is hexamethylene diamine, polyetheramine or a mixture thereof.

The structures of certain cross-linking agents are described in WO 2015/023961, the table on pages 13-15, which

are incorporated by reference.

Polyfunctional alcohols of use in this invention generally have at least two nucleophilic centers, *e.g.*, ethylene glycol, hexylene glycol, pentaerythritol, glucose, sorbitol, and 2-aminoethanol.

The range of polyfunctional amines, polyfunctional alcohols, or hybrid crosslinking agents can vary from 0.1% to 5% (*e.g.,* 0.2% to 3%, 0.2% to 2%, 0.5% to 2%, and 0.5% to 1%) by weight of the capsule delivery system. In one embodiment of the invention, the cross linking agent is added to the capsule reaction at a temperature of 0-55 °C (e.g., 10-50 °C , 15-45 °C, 20-40°C, and 22-35 °C).

By adding excess amount of a cross-linking agent, the polyurea/polyurethane formation is driven toward completion thereby reducing the amount of residual polyisocyanate. The reaction stoichiometry requires one amine/hydroxyl group per one isocyanate group. By way of illustration, when combining LUPRANATE M20 (having a molecular weight of 360 and isocyanate functionality of 2.7) and hexamethylenediamine (HMDA; having a molecular weight of 116.21 and amine functionality of 2), the stoichiometry of the system indicates that for each gram of HMDA, 2.23 grams of LUPRANATE is needed. The amount of amine will be in excess if more than one gram of HMDA is used per 2.23 grams of LUPRANATE M20. Using a crosslinker in excess, residual isocyanate amounts are reduced by at least 30%. After the capsules are formed, the free cross-link agent (e.g., hexamethylenediamine, amino-2-methyl-1-propanol, lysine, arginine, and histidine) can be present in the capsule slurry at a concentration of 20 ppm to 2 %. The amounts of the residual isocyanate and free cross-linking agent can be removed by washing the capsule slurry with water or carbonate/bicarbonate solution (e.g., sodium carbonate, potassium carbonate, sodium bicarbonate, and potassium bicarbonate).

In one embodiment of the invention, the cross linking agent is added to the capsule reaction at a temperature of 0-55 °C (e.g., 10-50 °C , 15-45 °C, 20-40°C, and 22-35 °C).

(iii) Catalysts. Catalysts suitable for use in the invention are metal carbonates, metal hydroxide, amino or organometallic compounds and include, for example, sodium carbonate, cesium carbonate, potassium carbonate, lithium hydroxide, 1,4-diazabicyclo[2.2.2]octane (*i.e.,* DABCO), N,N-dimethylaminoethanol, N,N-dimethylcyclohexylamine, bis-(2-dimethylaminoethyl) ether, N,N dimethylacetylamine, stannous octoate and dibutyltin dilaurate.

*Aminoplasts and Gelatin*

A representative process used for aminoplast encapsulation is disclosed in US 3,516,941 and US 2007/0078071, though it is recognized that many variations with regard to materials and process steps are possible. Another encapsulation process, *i.e.,* gelatin encapsulation, is disclosed in US 2,800,457. Both processes are discussed in the context of fragrance encapsulation for use in consumer products in US Patent Nos. 4,145,184 and 5,112,688 respectively. Polymer systems are well-known in the art and non-limiting examples of these include aminoplast capsules and encapsulated particles as disclosed in GB 2006709 A; the production of micro-capsules having walls comprising styrene-maleic anhydride reacted with melamine-formaldehyde precondensates as disclosed in US 4,396,670; an acrylic acid-acrylamide copolymer, cross-linked with a melamine-formaldehyde resin as disclosed in US 5,089,339; capsules composed of cationic melamine-formaldehyde condensates as disclosed in US 5,401,577; melamine formaldehyde microencapsulation as disclosed in US 3,074,845; amido-aldehyde resin in-situ polymerized capsules disclosed in EP 0 158 449 A1; etherified urea-formaldehyde polymer as disclosed in US 5,204,185; melamine-formaldehyde microcapsules as described in US 4,525,520; cross-linked oil-soluble melamine-formaldehyde precondensate as described in US 5,011,634; capsule wall material formed from a complex of cationic and anionic melamine-formaldehyde precondensates that are then cross-linked as disclosed in US 5,013,473; polymeric shells made from addition polymers such as condensation polymers, phenolic aldehydes, urea aldehydes or acrylic polymer as disclosed in US 3,516,941; urea-formaldehyde capsules as disclosed in EP 0 443 428 A2; melamine-formaldehyde chemistry as disclosed in GB 2 062 570 A; and capsules composed of polymer or copolymer of styrene sulfonic acid in acid of salt form, and capsules cross-linked with melamine-formaldehyde as disclosed in US 4,001,140.

*Urea-formaldehyde and melamine-formaldehyde Capsules*

Urea-formaldehyde and melamine-formaldehyde pre-condensate capsule shell wall precursors are prepared by means of reacting urea or melamine with formaldehyde where the mole ratio of melamine or urea to formaldehyde is in the range of from about 10:1 to about 1:6, preferably from about 1:2 to about 1:5. For purposes of practicing this invention, the resulting material has a molecular weight in the range of from 156 to 3000. The resulting material may be used 'as-is' as a cross-linking agent for the aforementioned substituted or un-substituted acrylic acid polymer or copolymer or it may be further reacted with a $C_1$-$C_6$ alkanol, *e.g.*, methanol, ethanol, 2-propanol, 3-propanol, 1-butanol, 1-pentanol or 1-hexanol, thereby forming a partial ether where the mole ratio of melamine/urea:formaldehyde:alkanol is in the range of 1:(0.1-6):(0.1-6). The resulting ether moiety-containing product may be used 'as-is' as a cross-linking agent for the aforementioned substituted or un-substituted acrylic acid polymer or copolymer, or it may be self-condensed to form

dimers, trimers and/or tetramers which may also be used as cross-linking agents for the aforementioned substituted or un-substituted acrylic acid polymers or co-polymers. Methods for formation of such melamine-formaldehyde and urea-formaldehyde pre-condensates are set forth in US Patent Nos. 3,516,846 and 6,261,483, and Lee et al. (2002) J. Microencapsulation 19, 559-569.

Examples of urea-formaldehyde pre-condensates useful in the practice of this invention are URAC 180 and URAC 186, trademarks of Cytec Technology Corp. of Wilmington, DE. Examples of melamine-formaldehyde pre-condensates useful in the practice if this invention, include, but are not limited to, CYMEL U-60, CYMEL U-64 and CYMEL U-65, trademarks of Cytec Technology Corp. of Wilmington, DE. It is preferable to use, as the precondensate for cross-linking, the substituted or un-substituted acrylic acid polymer or co-polymer. In practicing this invention, the range of mole ratios of urea-formaldehyde precondensate/melamine-formaldehyde pre-condensate to substituted/un-substituted acrylic acid polymer/co-polymer is in the range of from about 9:1 to about 1:9, preferably from about 5:1 to about 1:5 and most preferably from about 2:1 to about 1:2.

In one embodiment of the invention, microcapsules with polymer(s) composed of primary and/or secondary amine reactive groups or mixtures thereof and cross-linkers can also be used. See US 2006/0248665. The amine polymers can possess primary and/or secondary amine functionalities and can be of either natural or synthetic origin. Amine-containing polymers of natural origin are typically proteins such as gelatin and albumen, as well as some polysaccharides. Synthetic amine polymers include various degrees of hydrolyzed polyvinyl formamides, polyvinylamines, polyallyl amines and other synthetic polymers with primary and secondary amine pendants. Examples of suitable amine polymers are the LUPAMIN series of polyvinyl formamides available from BASF. The molecular weights of these materials can range from 10,000 to 1,000,000.

Urea-formaldehyde or melamine-formaldehyde capsules can also include formaldehyde scavengers, which are capable of binding free formaldehyde. When the capsules are for use in aqueous media, formaldehyde scavengers such as sodium sulfite, melamine, glycine, and carbohydrazine are suitable. When the capsules are aimed to be used in products having low pH, *e.g.*, fabric care conditioners, formaldehyde scavengers are preferably selected from beta diketones, such as beta-ketoesters, or from 1,3-diols, such as propylene glycol. Preferred beta-ketoesters include alkyl-malonates, alkyl aceto acetates and polyvinyl alcohol aceto acetates.

*Capsule formation aids*

Most capsule formation aids are used as dispersants (namely, emulsifiers or surfactants). They facilitate the formation of stable emulsions containing nano- or micro-sized oil drops to be encapsulated. Further, capsule formation aids improve the performance of the capsule delivery system by stabilizing capsules and/or their deposition to the target areas or releasing to the environment. Performance is measured by the intensity of the fragrance release during the pre-rub phase and post-rub. The pre-rub phase is the phase when the capsules have been deposited on the cloth, e.g., after a fabric softener containing capsules has been used during the wash cycle. The post-rub phase is after the capsules have been deposited and the capsules are broken by friction or other similar mechanisms.

In general, the amount of the capsule formation aid varies from 0.1 to 5% (e.g., 0.05 to 0.2 %, 0.5 to 4%, 0.2 to 2%, 1 to 2%, and 1% to 3%) by weight of the capsule composition.

In some embodiments, the capsule formation aid is a protective colloid or emulsifier including, e.g., maleic-vinyl copolymers such as the copolymers of vinyl ethers with maleic anhydride or acid, sodium lignosulfonates, maleic anhydride/styrene copolymers, ethylene/maleic anhydride copolymers, and copolymers of propylene oxide and ethylene oxide, polyvinylpyrrolidone (PVP), polyvinyl alcohols (PVA), sodium salt of naphthalene sulfonate condensate, carboxymethyl cellulose (CMC), fatty acid esters of polyoxyethylenated sorbitol, sodium dodecylsulfate, and any combination thereof.

Commercially available surfactants include, but are not limited to, sulfonated naphthalene-formaldehyde condensates such as MORWET D425 (naphthalene sulfonate, Akzo Nobel, Fort Worth, TX); partially hydrolyzed polyvinyl alcohols such as MOWIOLs, e.g., MOWIOL 3-83 (Air Products); ethylene oxide-propylene oxide block copolymers or poloxamers such as PLURONIC, SYNPERONIC or PLURACARE materials (BASF); sulfonated polystyrenes such as FLEXAN II (Akzo Nobel); ethylene-maleic anhydride polymers such as ZEMAC (Vertellus Specialties Inc.); and Polyquaternium series such as Polyquaternium 11 ("PQ11;" a copolymer of vinyl pyrrolidone and quaternized dimethylaminoethyl methacrylate; sold by BASF as LUVIQUAT PQ11 AT 1).

In other embodiments, the capsule formation aid is a processing aid such as hydrocolloids, which improve the colloidal stability of the slurry against coagulation, sedimentation and creaming. The term "hydrocolloid" refers to a broad class of water-soluble or water-dispersible polymers having anionic, cationic, zwitterionic or non-ionic character. Hydrocolloids useful in the present invention include, but are not limited to, polycarbohydrates, such as starch, modified starch, dextrin, maltodextrin, and cellulose derivatives, and their quaternized forms; natural gums such as alginate esters, carrageenan, xanthanes, agar-agar, pectines, pectic acid, and natural gums such as gum arabic, gum tragacanth and gum karaya, guar gums and quaternized guar gums; gelatine, protein hydrolysates and their quaternized forms; synthetic

polymers and copolymers, such as poly(vinyl pyrrolidone-co-vinyl acetate), poly(vinyl alcohol-co-vinyl acetate), poly((met)acrylic acid), poly(maleic acid), poly(alkyl(meth)acrylate-co-(meth)acrylic acid), poly(acrylic acid-co-maleic acid)copolymer, poly(alkyleneoxide), poly(vinylmethylether), poly(vinylether-co-maleic anhydride), and the like, as well as poly-(ethyleneimine), poly((meth)acrylamide), poly(alkyleneoxide-co-dimethylsiloxane), poly(amino dimethylsiloxane), and the like, and their quaternized forms.

The capsule formation aid may also be used in combination with CMC, polyvinylpyrrolidone, polyvinyl alcohol, alkylnaphthalenesulfonate formaldehyde condensates, and/or a surfactant during processing to facilitate capsule formation. Examples of surfactants that can be used in combination with the capsule formation aid include, but are not limited to, cetyl trimethyl ammonium chloride (CTAC), poloxamers such as PLURONICS (e.g., PLURONIC F127), PLURAFAC (e.g., PLURAFAC F127), or MIRANET-N, saponins such as QNATURALE (National Starch Food Innovation); or a gum Arabic such as Seyal or Senegal. In certain embodiments, the CMC polymer has a molecular weight range between about 90,000 Daltons to 1,500,000 Daltons, preferably between about 250,000 Daltons to 750,000 Daltons and more preferably between 400,000 Daltons to 750,000 Daltons. The CMC polymer has a degree of substitution between about 0.1 to about 3, preferably between about 0.65 to about 1.4, and more preferably between about 0.8 to about 1.0. The CMC polymer is present in the capsule slurry at a level from about 0.1% to about 2% and preferably from about 0.3% to about 0.7%. in other embodiments, polyvinylpyrrolidone used in this invention is a water-soluble polymer and has a molecular weight of 1,000 to 10,000,000. Suitable polyvinylpyrrolidone are polyvinylpyrrolidone K12, K15, K17, K25, K30, K60, K90, or a mixture thereof. The amount of polyvinylpyrrolidone is 2-50%, 5-30%, or 10-25% by weight of the capsule delivery system. Commercially available alkylnaphthalenesulfonate formaldehyde condensates include MORWET D-425, which is a sodium salt of naphthalene sulfonate condensate by Akzo Nobel, Fort Worth, TX.

*Chain Termination Agent*

Polymerization reactions for forming polyurea/polyurethane polymers can be terminated by adding a chain termination agent, *e.g.*, a monofunctional amine or alcohol. Further, a chain termination agent also reacts with isocyanate groups on the surface of the capsules, thus reduced/eliminated isocyanate groups. Examples of a chain termination agent include $C_1$-$C_{20}$ primary and secondary amines, $C_1$-$C_{20}$ alcohols, $C_1$-$C_{20}$ thiols, and any combination thereof.

*Active Materials*

The core of the capsules of the invention can include one or more active materials including, but not limited to, flavors and/or fragrance ingredients such as fragrance oils. Individual active materials that can be encapsulated include those listed in WO 2016049456, pages 38-50. These active material include flavor or fragrance ingredients, taste masking agents, taste sensates, malodor counteracting agents, vitamins, antibacterials, sunscreen actives, antioxidants, anti-inflammatory agents, anesthetics, analgesics, antifungal agents, antibiotics, anti-viral agents, anti-parasitic agents, anti-infectious and anti-acne agents, dermatological active ingredients, enzymes and co-enzymes, skin whitening agents, anti-histamines, chemotherapeutic agents, and insect repellents.

In addition to the active materials listed above, the products of this invention can also contain, for example, the following dyes, colorants or pigments: lactoflavin (riboflavin), beta-carotene, riboflavin-5'-phosphate, alpha-carotene, gamma-carotene, cantaxanthin, erythrosine, curcumin, quinoline yellow, yellow orange S, tartrazine, bixin, norbixin (annatto, orlean), capsanthin, capsorubin, lycopene, beta-apo-8'-carotenal, beta-apo-8'-carotenic acid ethyl ester, xantophylls (flavoxanthin, lutein, cryptoxanthin, rubixanthin, violaxanthin, rodoxanthin), fast carmine (carminic acid, cochineal), azorubin, cochineal red A (Ponceau 4 R), beetroot red, betanin, anthocyanins, amaranth, patent blue V, indigotine I (indigo-carmine), chlorophylls, copper compounds of chlorophylls, acid brilliant green BS (lissamine green), brilliant black BN, vegetable carbon, titanium dioxide, iron oxides and hydroxides, calcium carbonate, aluminum, silver, gold, pigment rubine BK (lithol rubine BK), methyl violet B, victoria blue R, victoria blue B, acilan brilliant blue FFR (brilliant wool blue FFR), naphthol green B, acilan fast green 10 G (alkali fast green 10 G), ceres yellow GRN, sudan blue II, ultramarine, phthalocyanine blue, phthalocayanine green, fast acid violet R. Further naturally obtained extracts (for example paprika extract, black carrot extract, red cabbage extract) can be used for coloring purposes. Goods results are also achieved with the colors named in the following, the so-called aluminum lakes: FD & C Yellow 5 Lake, FD & C Blue 2 Lake, FD & C Blue 1 Lake, Tartrazine Lake, Quinoline Yellow Lake, FD & C Yellow 6 Lake, FD & C Red 40 Lake, Sunset Yellow Lake, Carmoisine Lake, Amaranth Lake, Ponceau 4R Lake, Erythrosyne Lake, Red 2G Lake, Allura Red Lake, Patent Blue V Lake, Indigo Carmine Lake, Brilliant Blue Lake, Brown HT Lake, Black PN Lake, Green S Lake and mixtures thereof.

When the active material is a fragrance, it is preferred that fragrance ingredients within a fragrance having a ClogP of 0.5 to 15 are employed. For instance, the ingredients having a ClogP value between 0.5 to 8 (e.g., between 1 to 12, between 1.5 to 8, between 2 and 7, between 1 and 6, between 2 and 6, between 2 and 5, between 3 and 7) are 25 % or greater (e.g., 50 % or greater and 90 % or greater) by the weight of the fragrance.

In some embodiments, it is preferred that a fragrance having a weight-averaged ClogP of 2.5 and greater (e.g., 3 or greater, 2.5 to 7, and 2.5 to 5) is employed. The weight-averaged ClogP is calculated as follows:

$$ClogP = \{Sum\ [(Wi)(ClogP)i]\ \}/\{Sum\ Wi\ \},$$

in which Wi is the weight fraction of each fragrance ingredient and (ClogP)i is the ClogP of that fragrance ingredient.

As an illustration, it is preferred that greater than 60 weight percent, preferably greater than 80 and more preferably greater than 90 weight percent of the fragrance chemicals have ClogP values of greater than 2, preferably greater than 3.3, more preferably greater than 4, and even more preferably greater than 4.5.

In other embodiemnts, the ingredients having a ClogP value between 2 and 7 (e.g., between 2 and 6, and between 2 and 5) are 25 % or greater (e.g., 50 % or greater and 90 % or greater) by the weight of the fragrance. In still other embodiments, it is preferred that greater than 60 %, preferably greater than 80 % and more preferably greater than 90 % of the fragrance chemicals have Clog P values of greater than 3.3, preferably greater than 4 and most preferably greater than 4.5.

Those with skill in the art will appreciate that many fragrances can be created employing various solvents and fragrance chemicals. The use of a relatively low to intermediate ClogP fragrance ingredients will result in fragrances that are suitable for encapsulation. These fragrances are generally water-insoluble, to be delivered through the capsule systems of this invention onto consumer products in different stages such as damp and dry fabric. Without encapsulation, the free fragrances would normally have evaporated or dissolved in water during use, e.g., wash. Though high logP materials are generally well delivered from a regular (non-encapsulated) fragrance in a consumer product, they have excellent encapsulation properties and are also suitable for encapsulation for overall fragrance character purposes, very long-lasting fragrance delivery, or overcoming incompatibility with the consumer product, e.g., fragrance materials that would otherwise be instable, cause thickening or discoloration of the product or otherwise negatively affect desired consumer product properties.

In some embodiments, the amount of encapsulated active material is from 5 to 95% (*e.g.,* 20 to 90% and 40 to 85%) by weight of the capsule. The amount of the capsule wall is from 0.5% to 25% (e.g., 1.5 to 15% and 2.5 to 10%) also by weight of the capsule. In other embodiments, the amount of the encapsulated active material is from 15% to 99.5% (e.g., 50 to 98% and 30 to 95%) by weight of the capsule, and the amount of the capsule wall is from 0.5% to 85% (e.g., 2 to 50% and 5 to 70%) by weight of the capsule.

*Adjunct Materials*

In addition to the active materials, the present invention also contemplates the incorporation of adjunct materials including solvent, emollients, and core modifier materials in the core encapsulated by the capsule wall. Other adjunct materials are solubility modifiers, density modifiers, stabilizers, viscosity modifiers, pH modifiers, or any combination thereof. These modifiers can be present in the wall or core of the capsules, or outside the capsules in delivery system. Preferably, they are in the core as a core modifier.

The one or more adjunct material may be added in the amount of from 0.01% to 25% (e.g., from 0.5% to 10%) by weight of the capsule.

Suitable examples include those described in WO 2016/049456, pages 55-57 and US 2016/0158121, pages 15-18.

*Deposition Aids*

A capsule deposition aid from 0.01 to 25%, more preferably from 5 to 20% can be included by weight of the capsule. The capsule deposition aid can be added during the preparation of the capsules or it can be added after the capsules have been made.

These deposition aids are used to aid in deposition of capsules to surfaces such as fabric, hair or skin. These include anionically, cationically, nonionically, or amphoteric water-soluble polymers. Suitable deposition aids include polyquaternium-4, polyquaternium-5, polyquaternium-6, polyquaternium-7, polyquaternium-10, polyquaternium-16, polyquaternium-22, polyquaternium-24, polyquaternium-28, polyquaternium-39, polyquaternium-44, polyquaternium-46, polyquaternium-47, polyquaternium-53, polyquaternium-55, polyquaternium-67, polyquaternium-68, polyquaternium-69, polyquaternium-73, polyquaternium-74, polyquaternium-77, polyquaternium-78, polyquaternium-79, polyquaternium-80, polyquaternium-81, polyquaternium-82, polyquaternium-86, polyquaternium-88, polyquaternium-101, polyvinylamine, polyethyleneimine, polyvinylamine and vinylformamide copolymer, an acrylamidopropyltrimonium chloride/acrylamide copolymer, a methacrylamidopropyltrimonium chloride/acrylamide copolymer, and combinations thereof.

Other suitable deposition aids include those described in WO 2016049456, pages 13-27. Additional deposition aids are described in US 2013/0330292, US 2013/0337023, and US 2014/0017278.

*Capsule delivery system*

The capsule of this invention can be formulated into a capsule composition or delivery system for use in consumer products.

The capsule composition can be a slurry containing a capsule suspended in a solvent (*e.g.,* water). The capsule is typically present at a level 0.1 to 80% (*e.g.,* 1 to 65% and 5 to 45%) by weight of the capsule composition.

In some embodiments, the capsule and its slurry prepared in accordance with the present invention is subsequently purified. Purification can be achieved by washing the capsule slurry with water, *e.g.*, deionized or double deionized water, until a neutral pH is achieved. For the purposes of the present invention, the capsule suspension can be washed using any conventional method including the use of a separatory funnel, filter paper, centrifugation and the like. The capsule suspension can be washed one, two, three, four, five, six, or more times until a neutral pH, *e.g.*, pH 6-8 and 6.5-7.5, is achieved. The pH of the purified capsules can be determined using any conventional method including pH paper, pH indicators, or a pH meter.

A capsule suspension of this invention is "purified" in that it is 80%, 90%, 95%, 98% or 99% homogeneous to capsules. In accordance with the present invention, purity is achieved by washing the capsules until a neutral pH is achieved, which is indicative of removal of unwanted impurities and/or starting materials, *e.g.*, polyisocyanate, cross-linking agent and the like.

In certain embodiments of this invention, the purification of the capsules includes the additional step of adding a salt to the capsule suspension prior to the step of washing the capsule suspension with water. Exemplary salts of use in this step of the invention include, but are not limited to, sodium chloride, potassium chloride or bi-sulphite salts. See US 2014/0017287.

(ii) Spray drying. The delivery system can also be spray dried to a solid form. In a spray drying process, a spray dry carrier is added to a capsule delivery system to assist the removal of water from the slurry.

According to one embodiment, the spray dry carriers can be selected from the group consisting of carbohydrates such as chemically modified starches and/or hydrolyzed starches, gums such as gum arabic, proteins such as whey protein, cellulose derivatives, clays, synthetic water-soluble polymers and/or copolymers such as polyvinyl pyrrolidone, polyvinyl alcohol. The spray dry carriers may be present in an amount from 1 to 50%, more preferably from 5 to 20%.

Optionally, a free flow agent (anticaking agent) of silicas which may be hydrophobic (i.e. silanol surface treated with halogen silanes, alkoxysilanes, silazanes, siloxanes, etc. such as Sipernat D17, Aerosil R972 and R974 (available from Degussa), etc.) and/or hydrophilic such as Aerosil 200, Sipernat 22S, Sipernat 50S, (available from Degussa), Syloid 244 (available from Grace Davison), may be present from about 0.01% to about 10%, more preferable from 0.5% to about 5%.

Humectants and viscosity control/suspending agents can also be added to facilitate spray drying. These agents are disclosed in U.S. Patent Nos. 4,428,869, 4,464,271, 4,446,032, and 6,930,078. Details of hydrophobic silicas as a functional delivery vehicle of active materials other than a free flow/anticaking agent are disclosed in U.S. Patent Nos. 5,500,223 and 6,608,017.

The spray drying inlet temperature is in the range of 150 to 240 °C, preferably between 170 and 230 °C, more preferably between 190 and 220 °C.

As described herein, the spray-dried capsule delivery system is well suited for use in a variety of all dry (anhydrous) products: powder laundry detergent, fabric softener dryer sheets, household cleaning dry wipes, powder dish detergent, floor cleaning cloths, or any dry form of personal care products (e.g. shampoo powder, deodorant powder, foot powder, soap powder, baby powder), etc. Because of high fragrance and/or active agent concentration in the spray-dried products of the present invention, characteristics of the aforementioned consumer dry products will not be adversely affected by a small dosage of the spray-dried products.

The capsule delivery system can also be sprayed as a slurry onto a consumer product, *e.g.*, a fabric care product. By way of illustration, a liquid delivery system containing capsules is sprayed onto a detergent powder during blending to make granules. See US 2011/0190191. In order to increase fragrance load, water-absorbing material, such as zeolite, can be added to the delivery system.

Alternatively, granulates in a consumer product are prepared in a mechanical granulator in the presence of a granulation auxiliary such as non-acid water-soluble organic crystalline solids. See WO 2005/097962.

(iii) Additional components. The capsule delivery system can include one or more non-confined unencapsulated active materials from about 0.01% to about 50%, more preferably from about 5% to about 40%.

The capsule delivery system can also contain one or more other delivery system such as polymer-assisted delivery compositions (see US 8,187,580), fiber-assisted delivery compositions (US 2010/0305021), cyclodextrin host guest complexes (US 6,287,603 and US 2002/0019369), pro-fragrances (WO 2000/072816 and EP 0 922 084), and any combination thereof. The capsule delivery system can also contain one or more (*e.g.*, two, three, four, five or six more) different capsules including different capsules of this invention and other capsules such as such as aminoplasts, hydrogel, sol-gel, coascervate capsules, polyurea/polyurethane capsules, and melamine formaldehyde capsules. More exemplary delivery systems that can be incorporated are coascervate capsules, cyclodextrin delivery systems, and pro-perfumes.

Examples of additional components include those described in US 2016/0158121, pages 24 to 25.

Any compound, polymer, or agent discussed above can be the compound, polymer, or agent itself as shown above, or its salt, precursor, hydrate, or solvate. A salt can be formed between an anion and a positively charged group on the compound, polymer, or agent. Suitable anions include chloride, bromide, iodide, sulfate, nitrate, phosphate, citrate, methanesulfonate, trifluoroacetate, acetate, malate, tosylate, tartrate, fumurate, glutamate, glucuronate, lactate, glutarate, and maleate. Likewise, a salt can also be formed between a cation and a negatively charged group on the compound, polymer, or agent. Suitable cations include sodium ion, potassium ion, magnesium ion, calcium ion, and an ammonium cation (e.g., tetramethylammonium ion). A precursor can be ester and another suitable derivative, which, during the process of preparing a polyurea or polyurethane capsule composition of this invention, is capable of converting to the compound, polymer, or agent and being used in preparing the polyurea or polyurethane capsule composition. A hydrate refers to the compound, polymer, or agent that contains water. A solvate refers to a complex formed between the compound, polymer, or agent and a suitable solvent. A suitable solvent can be water, ethanol, isopropanol, ethyl acetate, acetic acid, and ethanolamine.

Certain compounds, polymers, and agents have one or more stereocenters, each of which can be in the R configuration, the S configuration, or a mixture. Further, some compounds, polymers, and agents possess one or more double bonds wherein each double bond exists in the E (trans) or Z (cis) configuration, or combinations thereof. The compounds, polymers, and agents include all possible configurational stereoisomeric, regioisomeric, diastereomeric, enantiomeric, and epimeric forms as well as any mixtures thereof. As such, lysine used herein includes L-lysine, D-lysine, L-lysine monohydrochloride, D-lysine monohydrochloride, lysine carbonate, and so on. Similarly, arginine includes L-arginine, D-arginine, L-arginine monohydrochloride, D-arginine monohydrochloride, arginine carbonate, arginine monohydrate, and etc. Guanidine includes guanidine hydrochloride, guanidine carbonate, guanidine thiocyanate, and other guanidine salts including their hydrates. Ornithine include L-ornithine and its salts/hydrates (e.g., monohydrochloride) and D-ornithine and its salts/hydrates (e.g., monohydrochloride).

***Applications.*** The delivery systems of the present invention are well-suited for use, without limitation, in the following products:

a) Household products

i. Liquid or Powder Laundry Detergents which can use the present invention include those systems described in U.S. Pat. Nos. 5,929,022, 5,916,862, 5,731,278, 5,565,145, 5,470,507, 5,466,802, 5,460,752, 5,458,810, 5,458,809, 5,288,431, 5,194,639, 4,968,451, 4,597,898, 4,561,998, 4,550,862, 4,537,707, 4,537,706, 4,515,705, 4,446,042, and 4,318,818.

ii. Unit Dose Pouches, Tablets and Capsules such as those described in EP 1 431 382 A1, US 2013/0219996 A1, US 2013/0284637 A1, and US 6,492,315. These unit dose formulations can contain high concentrations of a functional material (*e.g.,* 5-100% fabric softening agent or detergent active), fragrance (*e.g.,* 0.5-100%, 0.5-40%, and 0.5-15%), and flavor (*e.g.,* 0.1-100%, 0.1-40%, and 1-20%). They can contain no water to limit the water content as low as less than 30% (*e.g.,* less than 20%, less than 10%, and less than 5%).

iii. Scent Boosters such as those described in US 7,867,968, US 7,871,976, US 8,333,289, US 2007/0269651 A1, and US2014/0107010 A1.

iv. Fabric Care Products such as Rinse Conditioners (containing 1 to 30 weight % of a fabric conditioning active), Fabric Liquid Conditioners (containing 1 to 30 weight % of a fabric conditioning active), Tumble Drier Sheets, Fabric Refreshers, Fabric Refresher Sprays, Ironing Liquids, and Fabric Softener Systems such as those described in U.S. Pat. Nos. 6,335,315, 5,674,832, 5,759,990, 5,877,145, 5,574,179, 5,562,849, 5,545,350, 5,545,340, 5,411,671, 5,403,499, 5,288,417, 4,767,547 and 4,424,134.

Liquid fabric softeners/fresheners contains at least one fabric softening agent present, preferably at a concentration of 1 to 30% (e.g., 4 to 20%, 4 to 10%, and 8 to 15%). The ratio between the active material and the fabric softening agent can be 1 : 500 to 1 : 2 (e.g., 1 : 250 to 1 : 4 and 1 : 100 to 1 :8). As an illustration, when the fabric softening agent is 5% by weight of the fabric softener, the active material is 0.01 to 2.5%, preferably 0.02 to 1.25% and more preferably 0.1 to 0.63%. As another example, when the fabric softening agent is 20% by weight of the fabric softener, the active material is 0.04 to 10%, preferably 0.08 to 5% and more preferably 0.4

to 2.5%. The active material is a fragrance, malodor counteractant or mixture thereof. The liquid fabric softener can have 0.15 to 15% of capsules (e.g., 0.5 to 10%, 0.7 to 5%, and 1 to 3%). When including capsules at these levels, the neat oil equivalent (NOE) in the softener is 0.05 to 5% (e.g., 0.15 to 3.2%, 0.25 to 2%, and 0.3 to 1%). Suitable fabric softening agents include cationic surfactants. Non-limiting examples are quaternary ammonium compounds such as alkylated quaternary ammonium compounds, ring or cyclic quaternary ammonium compounds, aromatic quaternary ammonium compounds, diquaternary ammonium compounds, alkoxylated quaternary ammonium compounds, amidoamine quaternary ammonium compounds, ester quaternary ammonium compounds, and mixtures thereof. Fabric softening compositions, and components thereof, are generally described in US 2004/0204337 and US 2003/0060390. Suitable softening agents include esterquats such as Rewoquat WE 18 commercially available from Evonik Industries and Stepantex SP-90 commercially available from Stepan Company.

v. Liquid dish detergents such as those described in U.S. Pat. Nos. 6,069,122 and 5,990,065

vi. Automatic Dish Detergents such as those described in U.S. Pat. Nos. 6,020,294, 6,017,871, 5,968,881, 5,962,386, 5,939,373, 5,914,307, 5,902,781, 5,705,464, 5,703,034, 5,703,030, 5,679,630, 5,597,936, 5,581,005, 5,559,261, 4,515,705, 5,169,552, and 4,714,562

vii. All-purpose Cleaners including bucket dilutable cleaners and toilet cleaners

viii. Bathroom Cleaners

ix. Bath Tissue

x. Rug Deodorizers

xi. Candles

xii. Room Deodorizers

xiii. Floor Cleaners

xiv. Disinfectants

xv. Window Cleaners

xvi. Garbage bags/trash can liners

xvii. Air Fresheners including room deodorizer and car deodorizer, scented candles, sprays, scented oil air freshener, Automatic spray air freshener, and neutralizing gel beads

xviii. Moisture absorber

xix. Household Devices such as paper towels and disposable Wipes

xx. Moth balls/traps/cakes

b) Baby Care Products

i. Diaper Rash Cream/Balm

ii. Baby Powder

c) Baby Care Devices

i. Diapers

ii. Bibs

iii. Wipes

d) Oral Care Products. Tooth care products (as an example of preparations according to the invention used for oral care) generally include an abrasive system (abrasive or polishing agent), for example silicic acids, calcium carbonates, calcium phosphates, aluminum oxides and/or hydroxylapatites, surface-active substances, for example sodium lauryl sulfate, sodium lauryl sarcosinate and/or cocamidopropylbetaine, humectants, for example glycerol and/or sorbitol, thickening agents, for example carboxymethyl cellulose, polyethylene glycols, carrageenan and/or Laponite™, sweeteners, for example saccharin, taste correctors for unpleasant taste sensations, taste correctors for further, normally not unpleasant taste sensations, taste-modulating substances (for example inositol phosphate, nucleotides such as guanosine monophosphate, adenosine monophosphate or other substances such as sodium glutamate or 2-phenoxypropionic acid), cooling active ingredients, for example menthol derivatives, (for example L-menthyllactate, L-menthylalkylcarbonates, menthone ketals, menthane carboxylic acid amides), 2,2,2-trialkylacetic acid amides (for example 2,2-diisopropylpropionic acid methyl amide), icilin and icilin derivatives, stabilizers and active ingredients, for example sodium fluoride, sodium monofluorophosphate, tin difluoride, quaternary ammonium fluorides, zinc citrate, zinc sulfate, tin pyrophosphate, tin dichloride, mixtures of various pyrophosphates, triclosan, cetylpyridinium chloride, aluminum lactate, potassium citrate, potassium nitrate, potassium chloride, strontium chloride, hydrogen peroxide, flavorings and/or sodium bicarbonate or taste correctors.

i. Tooth Paste. An exemplary formulation as follows:

1.calcium phosphate 40-55%
2.carboxymethyl cellulose 0.8-1.2%
3.sodium lauryl sulfate 1.5-2.5%
4.glycerol 20-30%
5.saccharin 0.1-0.3%
6.flavor oil 1-2.5%
7. water q.s. to 100%
A typical procedure for preparing the formulation includes the steps of (i) mixing by a blender according to the foregoing formulation to provide a toothpaste, and (ii) adding a composition of this invention and blending the resultant mixture till homogeneous.

ii. Tooth Powder
iii. Oral Rinse
iv. Tooth Whiteners
v. Denture Adhesive

e) Health Care Devices

i. Dental Floss
ii. Toothbrushes
iii. Respirators
iv. Scented/flavored condoms

f) Feminine Hygiene Products such as Tampons, Feminine Napkins and Wipes, and Pantiliners
g) Personal Care Products: Cosmetic or pharmaceutical preparations, *e.g.*, a "water-in-oil" (W/O) type emulsion, an "oil-in-water" (O/W) type emulsion or as multiple emulsions, for example of the water-in-oil-in-water (W/O/W) type, as a PIT emulsion, a Pickering emulsion, a micro-emulsion or nano-emulsion; and emulsions which are particularly preferred are of the "oil-in-water" (O/W) type or water-in-oil-in-water (W/O/W) type. More specifically,

i. Personal Cleansers (bar soaps, body washes, and shower gels)
ii. In-shower conditioner
iii. Sunscreen ant tattoo color protection (sprays, lotions, and sticks)
iv. Insect repellants
v. Hand Sanitizer
vi. Antiinflammatory balms, ointments, and sprays
vii. Antibacterial ointments and creams
viii. Sensates
ix. Deodorants and Antiperspirants including aerosol and pump spray antiperspirant, stick antiperspirant, roll-on antiperspirant, emulsion spray antiperspirant, clear emulsion stick antiperspirant, soft solid antiperspirant, emulsion roll-on antiperspirant, clear emulsion stick antiperspirant, opaque emulsion stick antiperspirant, clear gel antiperspirant, clear stick deodorant, gel deodorant, spray deodorant, roll-on, and cream deordorant.
x. Wax-based Deodorant. An exemplary formulation as follows:

1.Parafin Wax 10-20%
2.Hydrocarbon Wax 5-10%
3.White Petrolatum 10-15%
4.Acetylated Lanolin Alcohol 2-4%
5.Diisopropyl Adipate 4-8%
6.Mineral Oil 40-60%
7.Preservative (as needed)
The formulation is prepared by (i) mixing the above ingredients, (ii) heating the resultant composition to 75 °C until melted, (iii) with stirring, adding 4% cryogenically ground polymer containing a fragrance while maintaining the temperature 75 °C, and (iv) stirring the resulting mixture in order to ensure a uniform suspension while a composition of this invention is added to the formulation.

xi. Glycol/Soap Type Deodorant. An exemplary formulation as follows:

1.Propylene Glycol 60-70%
2.Sodium Stearate 5-10%
3.Distilled Water 20-30%
4.2,4,4-Trichloro-2'- Hydroxy Diphenyl Ether, manufactured by the Ciba-Geigy Chemical Company and a Trademark of the Ciba-Geigy Chemical Company) 0.01-0.5%
The ingredients are combined and heated to 75 °C with stirring until the sodium stearate has dissolved. The resulting mixture is cooled to 40 °C followed by addition of a composition of this invention.

xii. Lotion including body lotion, facial lotion, and hand lotion
xiii. Body powder and foot powder
xiv. Toiletries
xv. Body Spray
xvi. Shave cream and male grooming products
xvii. Bath Soak
xviii. Exfoliating Scrub

h) Personal Care Devices

i. Facial Tissues
ii. Cleansing wipes

i) Hair Care Products

i. Shampoos (liquid and dry powder)
ii. Hair Conditioners (Rinse-out conditioners, leave-in conditioners, and cleansing conditioners)
iii. Hair Rinses
iv. Hair Refreshers
v. Hair perfumes
vi. Hair straightening products
vii. Hair styling products, Hair Fixative and styling aids
viii. Hair combing creams
ix. Hair wax
x. Hair foam, hair gel, nonaerosol pump spray
xi. Hair Bleaches, Dyes and Colorants
xii. Perming agents
xiii. Hair wipes

j) Beauty Care

i. Fine Fragrance - Alcoholic. Compositions and methods for incorporating fragrance capsules into alcoholic fine fragrances are described in US 4,428,869. Alcoholic fine fragrances may contain the following:

1.Ethanol (1-99%)
2.Water (0-99%)
3.A suspending aide including but not limited to: hydroxypropyl cellulose, ethyl cellulose, silica, microcrystalline cellulose, carrageenan, propylene glycol alginate, methyl cellulose, sodium carboxymethyl cellulose or xanthan gum (0.1-1%)
4.Optionally an emulsifier or an emollient may be included including but not limited to those listed above

ii. Solid Perfume
iii. Lipstick/lip balm
iv. Make-up cleanser
v. Skin care cosmetic such as foundation, pack, sunscreen, skin lotion, milky lotion, skin cream, emollients, skin whitening
vi. Make-up cosmetic including manicure, mascara, eyeliner, eye shadow, liquid foundation, powder foundation, lipstick and cheek rouge

k) Consumer goods packaging such as fragranced cartons, fragranced plastic bottles/boxes

l) Pet care products

i. Cat litter
ii. Flea and tick treatment products
iii. Pet grooming products
iv. Pet shampoos
v. Pet toys, treats, and chewables
vi. Pet training pads
vii. Pet carriers and crates

m) Confectionaries confectionery, preferably selected from the group consisting of chocolate, chocolate bar products, other products in bar form, fruit gums, hard and soft caramels and chewing gum

i. Gum

1.Gum base (natural latex chicle gum, most current chewing gum bases also presently include elastomers, such as polyvinylacetate (PVA), polyethylene, (low or medium molecular weight) polyisobutene (PIB), polybutadiene, isobutene-isoprene copolymers (butyl rubber), polyvinylethylether (PVE), polyvinylbutyether, copolymers of vinyl esters and vinyl ethers, styrene-butadiene copolymers (styrene-butadiene rubber, SBR), or vinyl elastomers, for example based on vinylacetate/vinyllaurate, vinylacetate/vinylstearate or ethylene/vinylacetate, as well as mixtures of the mentioned elastomers, as described for example in EP 0 242 325, U.S. Pat. No. 4,518,615, U.S. Pat. No. 5,093,136, U.S. Pat. No. 5,266,336, U.S. Pat. No. 5,601,858 or U.S. Pat. No. 6,986,709.) 20-25%
2.Powdered sugar 45-50%
3.glucose 15-17%
4.starch syrup 10-13%
5.plasticizer 0.1%
6.flavor 0.8-1.2%

The components described above were kneaded by a kneader according to the foregoing formulation to provide a chewing gum. Encapsulated Flavor or sensate is then added and blended till homogeneous.
ii. Breath Fresheners
iii. Orally Dissolvable Strips
iv. Chewable Candy
v. Hard Candy

n) Baked products, preferably selected from the group consisting of bread, dry biscuits, cakes and other cookies;
o) snack foods, preferably selected from the group consisting of baked or fried potato chips or potato dough products, bread dough products and corn or peanut-based extrudates;

i. Potato, tortilla, vegetable or multigrain chips
ii. Popcorn
iii. Pretzels
iv. Extruded stacks

p) Cereal Products preferably selected from the group consisting of breakfast cereals, muesli bars and precooked finished rice products
q) Alcoholic and non-alcoholic beverages, preferably selected from the group consisting of coffee, tea, wine, beverages containing wine, beer, beverages containing beer, liqueurs, schnapps, brandies, sodas containing fruit, isotonic beverages, soft drinks, nectars, fruit and vegetable juices and fruit or vegetable preparations; instant beverages, preferably selected from the group consisting of instant cocoa beverages, instant tea beverages and instant coffee beverages

i. Ready to drink liquid drinks
ii. Liquid Drink Concentrates
iii. Powder Drinks
iv. Coffee: Instant Cappucino

1.Sugar 30-40%
2.Milk Powder 24-35%
3.Soluble Coffee 20-25%
4.Lactose 1-15%
5.Food Grade Emulsifier 1-3%
6.Encapsulated Volatile Flavor 0.01-0.5%

v. Tea
vi. Alcoholic

r) Spice blends and consumer prepared foods

i. Powder gravy, sauce mixes
ii. Condiments
iii. Fermented Products

s)Ready to heat foods: ready meals and soups, preferably selected from the group consisting of powdered soups, instant soups, precooked soups

i. Soups
ii. Sauces
iii. Stews
iv. Frozen entrees

t) Dairy Products milk products, preferably selected from the group consisting of milk beverages, ice milk, yogurt, kefir, cream cheese, soft cheese, hard cheese, powdered milk, whey, butter, buttermilk and partially or fully hydrolyzed milk protein-containing products Flavored milk beverages

i. Yoghurt
ii. Ice cream
iii. Bean Curd
iv. Cheese

u) Soya protein or other soybean fractions, preferably selected from the group consisting of soya milk and products produced therefrom, soya lecithin-containing preparations, fermented products such as tofu or tempeh or products produced therefrom and soy sauces;
v) Meat products, preferably selected from the group consisting of ham, fresh or raw sausage preparations, and seasoned or marinated fresh or salt meat products
w) Eggs or egg products, preferably selected from the group consisting of dried egg, egg white and egg yolk
x) Oil-based products or emulsions thereof, preferably selected from the group consisting of mayonnaise, remoulade, dressings and seasoning preparations
y) fruit preparations, preferably selected from the group consisting of jams, sorbets, fruit sauces and fruit fillings; vegetable preparations, preferably selected from the group consisting of ketchup, sauces, dried vegetables, deep-frozen vegetables, precooked vegetables, vegetables in vinegar and preserved vegetables
z) Flavored pet foods.

The above-listed applications are all well known in the art. For example, fabric softener systems are described in US Patent Nos. 6,335,315, 5,674,832, 5,759,990, 5,877,145, 5,574,179; 5,562,849, 5,545,350, 5,545,340, 5,411,671, 5,403,499, 5,288,417, and 4,767,547, 4,424,134. Liquid laundry detergents include those systems described in U.S. Patent Nos. 5,929,022, 5,916,862, 5,731,278, 5,565,145, 5,470,507, 5,466,802, 5,460,752, 5,458,810, 5,458,809, 5,288,431, 5,194,639, 4,968,451, 4,597,898, 4,561,998, 4,550,862, 4,537,707, 4,537,706, 4,515,705, 4,446,042, and 4,318,818. Liquid dish detergents are described in U.S. Patent Nos. 6,069,122 and 5,990,065. Shampoo and conditioners that can employ the present invention include those described in US Patent Nos. 6,162,423, 5,968,286, 5,935,561, 5,932,203, 5,837,661, 5,776,443, 5,756,436, 5,661,118, 5,618,523, 5,275,755, 5,085,857, 4,673,568, 4,387,090 and 4,705,681. Automatic Dish Detergents are described in U.S. Pat. Nos. 6,020,294, 6,017,871, 5,968,881, 5,962,386, 5,939,373, 5,914,307, 5,902,781, 5,705,464, 5,703,034, 5,703,030, 5,679,630, 5,597,936, 5,581,005, 5,559,261, 4,515,705, 5,169,552, and 4,714,562.
All parts, percentages and proportions refer to herein and in the claims are by weight unless otherwise indicated.

The values and dimensions disclosed herein are not to be understood as being strictly limited to the exact numerical values recited. Instead, unless otherwise specified, each such value is intended to mean both the recited value and a functionally equivalent range surrounding that value. For example, a value disclosed as "50%" is intended to mean "about 50%."

The terms "g," "mg," and "μg" refer to "gram," "milligram," and "microgram," respectively. The terms "L" and "mL" refer to "liter" and "milliliter," respectively.

The terms "capsule" and "microcapsule" herein are used interchangeably.

The terms "polyfunctional isocyanate," "multifunctional isocyanate," and "polyisocyanate" all refer to a compound having two or more isocyanate (-NCO) groups.

The terms "polyfunctional amine," "multifunctional amine," and "polyamine" refers to a compound containing two or more primary or secondary amine groups. These terms also refers to a compound containing one or more primary/secondary amine groups and one or more hydroxyl groups (-OH).

The terms "polyfunctional alcohol," "multifunctional alcohol," "poly alcohol," and "polyol" refer to a compound having two or more hydroxyl groups.

The invention is described in greater detail by the following non-limiting examples. Without further elaboration, it is believed that one skilled in the art can, based on the description herein, utilize the present invention to its fullest extent. All publications cited herein are incorporated by reference in their entirety.

**EXAMPLE 1**

A capsule composition of this invention, *i.e.*, Composition 1, was prepared following the procedure described below using an amino-functionalized silicone and a cationically modified polyvinyl alcohol as co-dispersants.

More specifically, an oil phase was provided by mixing 192 g of a research fragrance accord, 48 g ofNEOBEE oil (commercially available Stepan, Chicago, Illinois), and 19.2 g of Takenate D110-N (a xylylene diisocyanate adduct polymer, commercially available from Mitsui Chemicals America, Rye Brook, New York). In a separate beaker, an aqueous solution (273 g) was prepared containing 1% Silamine T-SA (an amino-functionlized silicone, an aminoethylaminopropyl-based amine, commercially available from Siltech Corporation, Toronto, Canada) and 1% Selvol™ Ultralux AD (a cationically modified polymer, *i.e.*, vinyl amine/vinyl alcohol copolymer, commercially available from Sekisui Specialty Chemicals America, Dallas, Texas). The oil phase was emulsified into the aqueous phase to form an oil-in-water fragrance emulsion under high shearing (IKA - ULTRA TURRAX, T25 Basic) at 9500 rpm for three minutes. After the fragrance emulsion was heated to 35 °C, 50.4 g of 30% branched polyethylenimine, Lupasol WF (BASF) and 17.4 g of water were added under constant mixing with an overhead mixer. After 15 minutes of stirring at 35 °C, the resultant capsule slurry was cured at 55 °C for two hours and then cooled to room temperature to obtain Composition 1.

**EXAMPLE 2**

Another capsule composition of this invention, *i.e.,* Composition 2, was prepared following the same procedure as Composition described above except that Selvol™ Ultralux AD was replaced with a cationically modified polymer UCARE™ Polymer JR-30M (*i.e.*, polyquaternium-10, a quaternized hydroxyethyl cellulose commercially available from Dow).

**EXAMPLE 3**

A third microcapsule composition of this invention, *i.e.*, Composition 3, was prepared following the procedure described below.

An oil phase was prepared by mixing 180 g of a research fragrance accord, 45 g of NEOBEE oil (Stepan) and 19.2 g of polymethylene polyphenylpolyisocyanate (Lupranate M20, BASF). In a separate beaker, an aqueous phase (310 g) was prepared containing 1% Morwet D-425 (a sodium salt of naphthalene sulfonate condensate, AkzoNobel) and 1% Luviksol K90 (a polyvinylpyrrolidone, BASF). The oil phase was emulsified into the aqueous phase to form an oil-in-water fragrance emulsion under high shearing (IKA - ULTRA TURRAX, T25 Basic) at 9500 rpm for three minutes. The fragrance emulsion was heated to a 35 °C and 21.6 g of 40% hexamethylenediamine (Sigma-Aldrich) and 18.2 g of water weres added under constant mixing with an overhead mixer, followed by the addition of 6 g of an amino-functionalized silicone (sold under the trade name Silamine T-SA by Siltech). After 15 minutes of stirring at 35 °C, the capsule slurry was cured at 55 °C for two hours. The sample was then cooled to room temperature to obtain Composition 3.

**EXAMPLE 4**

A fourth microcapsule composition, *i.e.,* Composition 4 was prepared as follows. An oil phase was prepared

by mixing 192 g of a research fragrance accord, 48 g of NEOBEE oil (Stepan) and 19.2 g of polymethylene polyphenylpolyisocyanate (Lupranate M20, BASF). Aqueous phase (290 g) was prepared containing 1% Morwet D-425 (AkzoNobel) and 1% Luviksol K90 (BASF). The oil phase was then emulsified into the aqueous phase to form an oil-in-water fragrance emulsion under high shearing (IKA - ULTRA TURRAX, T25 Basic) at 9500 rpm for three minutes. The fragrance emulsion was heated to a 35 °C and 21.6 g of 40% hexamethylenediamine (Sigma-Aldrich) and 23.2 g of water was added under constant mixing with an overhead mixer. After the addition was then added 6 g of amino-functionalized silicone (Silamine 2972, bis-aminopropyl dimethicone, Siltech). After 15 minutes of stirring at 35 °C, the capsule slurry was cured at 55 °C for two hours and then cooled to room temperature to obtain Composition 4.

**EXAMPLE 5**

A fifth microcapsule composition of this invention, *i.e.,* Composition 5, was prepared following the same procedure as Composition 4 except that Silamine 2972 was replaced with Silquat J208-1B (8.6 g, an amino-functionalized silicone, PEG-8 Distearmonium Chloride PG-Dimethicone commercially available from Siltech).

**EXAMPLE 6**

A sixth microcapsule composition of this invention, *i.e*., Composition 6, was prepared following the same procedure as Composition 4 except that Silamine 2972 was replaced with Silquat AD (15 g, an amino-functionalized silicone Silicone Quaternium-8 commercially available from Siltech).

**EXAMPLE 7**

A silica-polyurea hybrid microcapsule composition of this invention, *i.e*., Composition 7, was prepared as follows.

An aqueous phase was prepared by mixing 30 g of 10% Ultalux AD (Selvol), 100 g of 3% UCARE™ Polymer JR-30M (Dow), 3 g of Silamine 2972 (Siltech) and 160.8 g of water. In a separate container, 192 g of a research fragrance accord was mixed with 48 g of NEOBEE oil (Stepan), 12.08 g of tetraorthosilicate (Evonik) and 11.52 g of a xylylene diisocyanate adduct polymer (Takenate D110-N, Mitsui Chemicals) to prepare an oil phase. The oil phase was then emulsified into the aqueous phase under high shearing (IKA - ULTRA TURRAX, T25 Digital Basic) at 8000 rpm for three minutes to form an oil-in-water fragrance emulsion. The fragrance emulsion was then heated to 35 °C and 5.04 g of branched polyethylenimine (BASF) and 37.56 g of water was added while stirred at 300 rpm with the overhead mixer. After 5 minutes of stirring at 35 °C, the slurry was then cured at 55 °C for two hours. Afterwards, the sample was cooled to room temperature to obtain Composition 7.

**EXAMPLE 8**

A silica-polyurea hybrid microcapsule composition of this invention, *i.e.,* Composition 8, was prepared as follows.

An aqueous phase was prepared by mixing 30 g of 10% Ultalux AD (Selvol), 100 g of 3% UCARE™ Polymer JR-30M (Dow), 3 g of Silamine 2972 (Siltech) and 160.8 g of water. In a separate container, 192 g of a research fragrance accord was mixed with 48 g of NEOBEE oil (Stepan), and 11.52 g of Takenate D110-N to prepare an oil phase. The oil phase was then emulsified into the aqueous phase under high shearing (IKA - ULTRA TURRAX, T25 Digital Basic) at 8000 rpm for three minutes to form an oil-in-water fragrance emulsion. The fragrance emulsion was then heated to 35 °C and 5.04 g of branched polyethylenimine (BASF), 37.56 g of water and 12.08 g of tetraorthosilicate (Evonik) were added while stirred at 300 rpm with the overhead mixer. After 5 minutes of stirring at 35 °C, the slurry was then cured at 55 °C for two hours. Afterwards, the sample was cooled to room temperature to obtain Composition 8.

**COMPARATIVES 1', 2', and 3'**

Comparative 1' was prepared following the same procedure as Composition 1 except that the aqueous phase was prepared by dissolving 30 g of 10% polystyrene sulfonate (Flexan II, AkzoNobel) and 60 g of 1% carboxymethyl cellulose (Walocel 50000 PA, Dow) in 200.4 g of water.

Comparative 2' was prepared as follows. An oil phase was prepared by mixing 192 g of a research fragrance accord, 48 g of NEOBEE oil (Stepan) and 19.2 g of polymethylene polyphenylpolyisocyanate (Lupranate M20, BASF). In a separate beaker, 319.2 g of an aqueous phase was prepared containing 0.9% Morwet D-425 (AkzoNobel) and 0.9% Luviksol K90 (BASF). The oil phase was then emulsified with the aqueous phase to form an oil-in-water fragrance emulsion under high shearing (IKA ULTRA TURRAX, T25 Basic) at 9500 rpm for 3 minutes. The fragrance emulsion was heated to 35 °C and 21.6 g of 40% hexamethylenediamine (Sigma-Aldrich) was added under constant mixing with an overhead mixer. After 15 minutes at 35 °C, the capsule slurry was cured at 55 °C for two hours and then cooled to

room temperature to obtain Comparative 2'.

Comparative 3' was prepared as follows. An aqueous phase was first obtained by dissolving 3 g of Flexan II (AkzoNobel), 0.6 g of CMC (Walocel CRT 50000 PA, commercially available from Dow) in 286.8 g of water. In a separate container, 192 g of a research fragrance accord was mixed with 48 g of NEOBEE oil (Stepan) and 19.2 g of tolylene-2,4-diisocyanate (Takenate D110-N, Mitsui Chemicals) to create an oil phase, which was consequently emulsified into the aqueous phase under high shearing (IKA - ULTRA TURRAX, T25 Digital Basic) at 9400 rpm for three minutes to form an oil-in-water fragrance emulsion. The fragrance emulsion was then heated to 35 °C and 50.4 g of branched polyethylenimine (BASF) was added while stirred at 300 rpm with the overhead mixer. After 5 minutes of stirring at 35 °C, the slurry was then cured at 55 °C for two hours and cooled to room temperature to obtain Comparative 3'.

**STABILITY PERFORMANCE**

The slurry viscosity of Compositions 1-6 and Comparatives 1' and 2' was measured using a Haake RheoStress 600. As shown in Table 1, it was surprisingly found that Compositions 1 and 3-6 all showed very low viscosity as well very fluid profile with minimal separation and caking. While the viscosity of Composition 2 was much higher, due to the viscous nature of the quaternized polysaccharide co-dispersant, the slurry remained very fluid with minimal separation and caking. None of Compositions 1-6 showed large separation and caking during storage. By contrast, the resulting slurry of Comparatives 2' was heavily aggregated with a very high viscosity beyond the instrument measurement limit. Comparative 1' showed a low stability initially, and overtime, large separation with thick caking was observed, preventing immediate remixing of the slurry.

TABLE 1.

| **EXAMPLES** | **Viscosity, 21 sec$^{-1}$** |
|---|---|
| COMPOSITION 1 | 103 |
| COMPOSITION 2 | 2947 |
| COMPOSITION 3 | 43 |
| COMPOSITION 4 | 68 |
| COMPOSITION 5 | 92 |
| COMPOSITION 6 | 91 |
| COMPARATIVE 1' | 465 |
| COMPARATIVE 2' | Immeasurable |

**SENSORY PERFORMANCE**

The performance of Compositions 2, 3, 7, and 8, and Comparatives 1'-3' was evaluated in a fabric conditioner base. More specifically, each composition was separately blended into a model fabric conditioner base at 1% fragrance oil equivalence. The resulting fabric conditioner was applied to a standard US washing machine protocol with towels as described in US 8299011. In the first sequence, damp towels were evaluated freshly out of the wash on a tray by sensory evaluation by a panel of judges. The towels were then line-dried for 24 hours followed by another sensory evaluation pre- and post-rubbing the towels at dry. The fragrance intensity was rated on a scale ranging from 0 to 10. A numerical value of 5 indicated the towel producing a strong intensity, while a value of 10 indicated the towel generating a very strong smell.

Composition 2 and Comparative 2' contained fragrance accord No Limit (International Flavors and Fragrances, Union Beach, New Jersey).

Composition 3 and Comparative 1' contained fragrance accord Greenfields (International Flavors and Fragrances, Union Beach, New Jersey).

Compositions 7 and 8 and Comparative 3' contained fragrance accord Fruity Bomb (International Flavors and Fragrances, Union Beach, New Jersey).

Table 2 below shows the fragrance intensity for the compositions at three stages of the fabric conditioning application, namely, Damp, Dry pre-rub, and Dry post-rub.

TABLE 2.

| Composition | Fabric Conditioner (Fragrance Intensity) | | |
|---|---|---|---|
| | Damp | Dry pre-rub | Dry post-rub |
| 2 | 2.15 | 1.14 | 6.07 |
| COMPARATIVE 2’ | 2.05 | 0.93 | 5.43 |
| 3 | 4.07 | 2.25 | 5.40 |
| COMPARATIVE 1’ | 3.08 | 1.70 | 3.20 |
| 7 | 3.00 | 1.58 | 5.92 |
| 8 | 4.10 | 2.67 | 5.92 |
| COMPARITIVE 3’ | 2.70 | 1.42 | 4.17 |

As shown in Table 2, the amino-functionalized silicones unexpectedly enhanced the dry post-rub intensity for Composition 2 as compared to Comparative 2’.

As to Composition 3, the amino-functionalized silicone unexpectedly enhanced the performance in all three stages as compared to Comparative 1’.

Similarly, the amino-functionalized silicone unexpectedly enhanced the performance in all three stages for Compositions 7 and 8 as compared to Comparative 3’.

**Capsule Performance from Liquid Detergent.** The performance of Compositions 2, 7 and 8 and Comparatives 2’ and 3’ was evaluated in a liquid detergent base. Composition 2 and Comparative 2’ contained the same fragrance (fragrance No Limit). Compositions 7 and 8 and Comparative 3’ contained the same fragrance (fragrance Fruity Bomb). Each of Composition 2 and Comparative 2’ was separately blended into a model liquid detergent base at 0.5% fragrance oil equivalence. The resulting liquid detergent was applied to a standard EU washing machine protocol with towels as described in US 8299011. In the first sequence, damp towels were evaluated freshly out of the wash on a tray by a panel of judges. The towels were then line-dried for 24 hours followed by another sensory evaluation pre- and post-rubbing the towels at dry. The fragrance intensity was rated on a scale ranging from 0 to 10 at. A numerical value of 5 indicated the towel producing a strong intensity, while a value of 10 indicated the towel generating a very strong smell.

TABLE 3.

| Composition | Fragrance Intensity | | |
|---|---|---|---|
| | Damp | Dry pre-rub | Dry post-rub |
| 2 | 3.03 | 1.00 | 4.07 |
| COMPARATIVE 2’ | 3.21 | 0.64 | 3.64 |
| 7 | 4.07 | 1.20 | 4.40 |
| 8 | 5.14 | 2.80 | 5.60 |
| COMPARATIVE 3’ | 3.36 | 0.80 | 2.70 |

As shown in Table 3 above, the amino-functionalized silicones unexpectedly enhanced the dry pre-rub and post-rub intensities for Compositions 2, 7, and 8.

## Microcapsule Composition Performance in a Shampoo

Compositions 7 and 8, and Comparative 3’ were also evaluated in a shampoo base. Each composition was blended into an un-fragranced shampoo base (commercially available from Magick Botanical) at 1% fragrance oil equivalent and at high shear, 4000-6000 rpm for 1 - 2 minutes. The sample thus prepared (2 g) was added to 2 bundles of hair swatch (8 strands) that was wetted under water, with excess water squeezed lightly. The swatches were then rinsed under a stream of water (38 °C, 1 gallon/minute) for 45 seconds. Excess water from hair was removed. Hair swatches were then line-dried for 24 hours followed by sensory evaluation by a panel of judges. The fragrance intensity was rated on a scale ranging from 0 to 10. A numerical value of 5 indicated the hair swatches produced a strong intensity, while a value of 10 indicated the hair swatches generated a very strong smell. One hair swatch was evaluated without brushing with a comb to obtain the pre-brush fragrance intensity and the other was used to obtain the post-brush fragrance intensity after brushing it with a standard hair comb. The results are shown in Table 4 below.

TABLE 4

| Composition | Fragrance Intensity | |
|---|---|---|
| | Dry pre-rub | Dry post-rub |
| 7 | 1.92 | 5.08 |
| 8 | 2.58 | 5.42 |
| COMPARITIVE 3' | 1.83 | 5.08 |

As shown in Table 4 above, Composition 7 had a greater fragrance intensity at dry pre-rub as compared to Comparative 3'. Composition 8 had a greater fragrance intensity at both dry pre-rub and dry post-rub.

**Microcapsule Composition Performance in a Hair Conditioner**

Compositions 7 and 8 and Comparative 3' were further evaluated in a hair conditioner base. Each composition was blended into a un-fragranced hair conditioner base (commercially available from Magick Botanicals) at 1% fragrance oil equivalent and at high shear, 4000-6000 rpm for 1 - 2 minutes. The sample thus prepared (2 g) was added to 2 bundles of hair swatches (8 strands) that were wetted under water, with excess water squeezed lightly. The swatches were then rinsed under a stream of water (38 °C, 1 gal/min) for 45 seconds. Excess water from hair was removed. Hair swatches were then line-dried for 24 hours followed by sensory evaluation by a panel of judges. The fragrance intensity was rated on a scale ranging from 0 to 10. A numerical value of 5 indicated the hair swatches produced a strong intensity, while a value of 10 indicated the hair swatches generated a very strong smell. One hair swatch was evaluated without brushing with a comb to obtain the pre-brush fragrance intensity and the other was used to obtain the post-brush fragrance intensity after brushing it with a comb. The results are shown in Table 5 below.

TABLE 5.

| Composition | Fragrance Intensity | |
|---|---|---|
| | Dry pre-rub | Dry post-rub |
| 7 | 1.57 | 4.07 |
| 8 | 2.14 | 4.50 |
| COMPARATIVE 3' | 1.50 | 2.64 |

As shown in Table 5 above, Composition 7 had a greater fragrance intensity at dry post-rub as compared to Comparative 3'. Composition 8 had a greater fragrance intensity at both dry pre-rub and dry post-rub.

**OTHER EMBODIMENTS**

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

Indeed, to achieve the purpose of preparing a capsule and a delivery system containing the capsule, one skilled in the art can choose different sol-gel precursors, aminopolysiloxane, and other wall polymer precursors, cross-linking agents, and/or capsule formation aids/catalysts, varying the concentrations of these wall-forming materials and/or catalysts to achieve desirable organoleptic or release profiles in a consumable product. Further, the ratios among their wall-forming materials, capsule forming aids, adjuvents, core modifiers, active materials, and catalysts can also be determined by a skilled artisan without undue experimentation. A skilled person can also choose a suitable stabilizing agent and determine its concentration in a capsule composition and final product.

From the above description, a skilled artisan can easily ascertain the essential characteristics of the present invention, and without departing from the spirit and scope thereof, can make various changes and modifications of the invention to adapt it to various usages and conditions. Thus, other embodiments are also within the claims.

**Claims**

**1.** A microcapsule composition comprising a microcapsule and an aminopolysiloxane, wherein

the microcapsule has an oil core containing an active material and a capsule wall encapsulating the oil core,
the microcapsule has a particle size of 0.1 to 1000 microns, and
the microcapsule wall is formed of an encapsulating polymer.

**2.** The microcapsule composition of claim 1, wherein the encapsulating polymer is a polyacrylate, polyurea, polyurethane, polyacrylamide, polyester, polyether, polyamide, poly(acrylate-co-acrylamide), starch, silica, gelatin and gum Arabic, alginate, chitosan, polylactide, poly(melamine-formaldehyde), poly(urea-formaldehyde), or combination thereof; and the active material is a fragrance, pro-fragrance, flavor, malodor counteractive agent, vitamin or derivative thereof, anti-inflammatory agent, fungicide, anesthetic, analgesic, antimicrobial active, anti-viral agent, anti-infectious agent, anti-acne agent, skin lightening agent, insect repellant, animal repellent, vermin repellent, emollient, skin moisturizing agent, wrinkle control agent, UV protection agent, fabric softener active, hard surface cleaning active, skin or hair conditioning agent, flame retardant, antistatic agent, nanometer to micron size inorganic solid, polymeric or elastomeric particle, taste modulator, cell, probiotic, or combination thereof.

**3.** The microcapsule composition of claim 1 or 2, further comprising a polyvinyl alcohol, polystyrene sulfonate, carboxymethyl cellulose, sodium polystyrene sulfonate, alkylnaphthalenesulfonate formaldehyde condensate, polyvinylpyrrolidone, copolymer of vinyl pyrrolidone and quaternized dimethylaminoethyl methacrylate, or combination thereof, wherein
the encapsulating polymer is a polyurea, the polyurea is a reaction product of a polyfunctional isocyanate and a polyfunctional amine;
the polyfunctional isocyanate is an aromatic polyfunctional isocyanate, aliphatic polyfunctional isocyanate, or combination thereof;
the aromatic polyfunctional isocyanate contains a phenyl, tolyl, xylyl, naphthyl, or diphenyl moiety, or a combination thereof;
the aliphatic polyfunctional isocyanate contains a trimer of hexamethylene diisocyanate, a trimer of isophorone diisocyanate, a biuret of hexamethylene diisocyanate, or a combination thereof; and
the polyfunctional amine contains hexamethylenediamine, hexaethylenediamine, ethylenediamine, 1,3-diaminopropane, 1,4-diamino-butane, diethylenetriamine, pentaethylenehexamine, 1,6-diaminohexane, hydrazine, 1,4-diaminocyclohexane and 1,3-diamino-1-methylpropane, bis(3-aminopropyl)amine, bis(hexamethylene)triamine, tris(2-aminoethyl)amine, triethylene-tetramine, N,N'-bis(3-aminopropyl)-1,3-propanediamine, tetraethylenepentamine, branched polyethylenimine, chitosan, nisin, gelatin, 1,3-diamino-guanidine, 1,1-dimethylbiguanide, guanidine, arginine, lysine, ornithine, histidine, amino-2-methyl-1-propanol, or a combination thereof.

**4.** The microcapsule composition of claim 3, wherein the aromatic polyfunctional isocyanate is selected from the group consisting of polymeric methylene diphenyl diisocyanate, polyisocyanurates of toluene diisocyanate, trimethylol propane-adducts of toluene diisocyanate, trimethylol propane-adducts of xylylene diisocyanate, and combinations thereof; and the aliphatic polyfunctional isocyanate is selected from the group consisting of dimers, biurets, symmetric trimers, asymmetric trimers of hexamethylene diisocyanate, and combinations thereof.

**5.** The microcapsule composition of claim 3 or 4, wherein each of the alkylnaphthalenesulfonate formaldehyde condensate and polyvinylpyrrolidone, independently, is present at 0.1 to 5% by weight of the microcapsule composition, and the ratio between the alkylnaphthalenesulfonate formaldehyde condensate and polyvinylpyrrolidone is 10 : 1 to 1 : 10.

**6.** The microcapsule composition of any one of the preceding claims, further comprising a cationically modified polyvinyl alcohol or quaternized polysaccharide.

**7.** The microcapsule composition of any one of the preceding claims, wherein the aminopolysiloxane has a structure of Formula I:

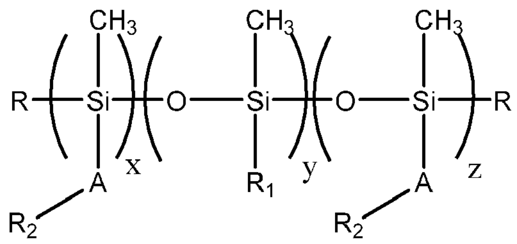

Formula I

wherein

R is $C_1$-$C_6$ alkyl, $C_1$-$C_6$ alkoxy, or -O-Si($CH_3$)$_3$;
$R_1$ is $C_1$-$C_6$ alkyl, $C_1$-$C_6$ alkoxy, -($OCH_2CH_2$)$_m$-H, or -($OCH_2CH_2CH_2$)$_n$-H, in which each of m and n, independently, is an integer from 0 to 10;
$R_2$ is NH2, NHR', or NR'R", in which each of R' and R', independently, is a $C_1$-$C_6$ alkyl;
A is $C_1$-$C_6$ alkyl, $C_1$-$C_6$ alkoxy, -($OCH_2CH_2$)$_m$-H, or -($OCH_2CH_2CH_2$)$_n$-H;
x is 0 to 2000;
y 1-2000; and
z is 1-2000.

**8.** The microcapsule composition of any one of the preceding claims, further comprising a deposition aid that is polyquaternium-4, polyquaternium-5, polyquaternium-6, polyquaternium-7, polyquaternium-10, polyquaternium-16, polyquaternium-22, polyquaternium-24, polyquaternium-28, polyquaternium-39, polyquaternium-44, polyquaternium-46, polyquaternium-47, polyquaternium-53, polyquaternium-55, polyquaternium-67, polyquaternium-68, polyquaternium-69, polyquaternium-73, polyquaternium-74, polyquaternium-77, polyquaternium-78, polyquaternium-79, polyquaternium-80, polyquaternium-81, polyquaternium-82, polyquaternium-86, polyquaternium-88, polyquaternium-101, polyvinylamine, polyethyleneimine, polyvinylamine and vinylformamide copolymer, an acrylamidopropyltrimonium chloride/acrylamide copolymer, a methacrylamidopropyltrimonium chloride/acrylamide copolymer, or a mixture thereof.

**9.** The microcapsule composition of any one of the preceding claims, wherein the encapsulating polymer contains a first polymer and a second polymer, the ratio between the first polymer and the second polymer is 1 : 10 to 10 : 1, the first polymer is a sol-gel polymer, and the second polymer is polyacrylate, polyacrylamide, poly(acrylate-co-acrylamide), polyurea, polyurethane, starch, gelatin and gum Arabic, poly(melamine-formaldehyde), poly(urea-formaldehyde), or a combination thereof, and optionally
wherein the first polymer is a silica gel or polyalkylsiloxane and the second polymer is a polyurea polymer.

**10.** A method of preparing the microcapsule composition of any one of the preceding claims, the method comprising:

(a) providing an oil phase having an active material, a first polymer precursor, and a second polymer precursor,
(b) providing an aqueous phase having an aminopolysiloxane,
(c) emulsifying the oil phase into the aqueous phase to form an oil-in-water emulsion,
(d) causing the formation of a capsule having an oil core that contains the active material and a capsule wall that is formed of the first polymer precursor and a second polymer precursor, and
(e) curing the capsule to obtain the microcapsule composition,

wherein the first polymer precursor is a sol-gel precursor, and the second polymer precursor is a acrylate monomer, acrylamide monomer, polyfunctional isocyanate, starch, gelatin-gum arabic, melamine-formaldehyde precondensate, urea-formaldehyde precondensate, or a combination thereof.

**11.** A method of preparing a microcapsule composition of any one of claims 1 to 9, the method comprising:

(a) providing an oil phase having an active material and a second polymer precursor,
(b) providing an aqueous phase having an aminopolysiloxane,
(c) emulsifying the oil phase into the aqueous phase to form an oil-in-water emulsion,
(d) adding a first polymer precursor to the oil-in-water emulsion,

(e) causing the formation of a capsule having an oil core that contains the active material and a capsule wall that is formed of the first polymer precursor and a second polymer precursor, and
(f) curing the capsule to obtain the microcapsule composition,

wherein the first polymer precursor is a sol-gel precursor, and the second polymer precursor is a acrylate monomer, acrylamide monomer, polyfunctional isocyanate, starch, gelatin-gum arabic, melamine-formaldehyde precondensate, urea-formaldehyde precondensate, or a combination thereof.

**12.** The method of claim 10 or 11, optionally comprising one or more of the following steps:

(c-1) adding an activation agent to the oil-in-water emulsion;
(e-2) spray drying the capsule composition to obtain a capsule composition in a powder form; and
(f) adding a rheology modifier to the microcapsule composition.

**13.** The method of claim 12, wherein the first polymer precursor is tetramethyl orthosilicate, tetraethyl orthosilicate, or a combination thereof; the second polymer precursor is a polyfunctional isocyanate; the activation agent is a polyfunctional amine; the aqueous phase further contains a cationically modified polyvinyl alcohol or quaternized polysaccharide; the capsule is cured at 40 to 250 °C for 10 minutes to 5 hours; and the rheology modifier is selected from the group consisting of alkali-swellable anionic acrylic polymer emulsion, anionic hydrophobically modified alkali-soluble acrylic polymer emulsion, anionic acrylic copolymer emulsion, hydrophobically-modified ethoxylated urethane, xanthan gum, carrageenan, gellan, pectin, hydroxyethyl cellulose, sodium carboxymethyl cellulose, guar, sodium alginate, fully exfoliated smectite clays, and combinations thereof.

**14.** A consumer product comprising the microcapsule composition of any one of claims 1 to 9, wherein the consumer product is a shampoo, hair conditioner, bar soap, liquid detergent, powder detergent, fabric conditioner, or fabric refresher.

**15.** Use of a microcapsule composition as defined in any one of claims 1 to 9, to deliver fragrance from a fabric at both the damp and dry stages of a laundry application.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011154893 A
- WO 2012107323 A
- US 20110077188 A
- US 5635211 A
- US 6586107 B
- US 6797670 B
- US 20140044760 A
- US 9044732 B
- US 20140287008 A
- US 20140044761 A
- US 20110033513 A
- US 20070138673 A
- US 20150252312 A
- US 20160193122 A
- US 20150203787 A
- US 8242215 B
- WO 2007141310 A
- US 20060252668 A
- US 20100326614 A
- US 6465416 B
- DE 10021165
- WO 2004054362 A
- WO 2015023961 A
- EP 0148149 A
- EP 0017409 B1
- US 4417916 A
- US 4124526 A
- US 5583090 A
- US 6566306 B
- US 6730635 B
- US 9008468 W
- WO 9213450 A
- US 4681806 A
- US 4285720 A
- US 6340653 B
- US 3516941 A
- US 20070078071 A
- US 2800457 A
- US 4145184 A
- US 5112688 A
- GB 2006709 A
- US 4396670 A
- US 5089339 A
- US 5401577 A
- US 3074845 A
- EP 0158449 A1
- US 5204185 A
- US 4525520 A
- US 5011634 A
- US 5013473 A
- EP 0443428 A2
- GB 2062570 A
- US 4001140 A
- US 3516846 A
- US 6261483 B
- US 20060248665 A
- WO 2016049456 A
- US 20160158121 A
- US 20130330292 A
- US 20130337023 A
- US 20140017278 A
- US 20140017287 A
- US 4428869 A
- US 4464271 A
- US 4446032 A
- US 6930078 B
- US 5500223 A
- US 6608017 B
- US 20110190191 A
- WO 2005097962 A
- US 8187580 B
- US 20100305021 A
- US 6287603 B
- US 20020019369 A
- WO 2000072816 A
- EP 0922084 A
- US 5929022 A
- US 5916862 A
- US 5731278 A
- US 5565145 A
- US 5470507 A
- US 5466802 A
- US 5460752 A
- US 5458810 A
- US 5458809 A
- US 5288431 A
- US 5194639 A
- US 4968451 A
- US 4597898 A
- US 4561998 A
- US 4550862 A
- US 4537707 A
- US 4537706 A
- US 4515705 A
- US 4446042 A
- US 4318818 A
- EP 1431382 A1
- US 20130219996 A1

- US 20130284637 A1
- US 6492315 B
- US 7867968 B
- US 7871976 B
- US 8333289 B
- US 20070269651 A1
- US 20140107010 A1
- US 6335315 B
- US 5674832 A
- US 5759990 A
- US 5877145 A
- US 5574179 A
- US 5562849 A
- US 5545350 A
- US 5545340 A
- US 5411671 A
- US 5403499 A
- US 5288417 A
- US 4767547 A
- US 4424134 A
- US 20040204337 A
- US 20030060390 A
- US 6069122 A
- US 5990065 A
- US 6020294 A
- US 6017871 A
- US 5968881 A
- US 5962386 A
- US 5939373 A
- US 5914307 A
- US 5902781 A
- US 5705464 A
- US 5703034 A
- US 5703030 A
- US 5679630 A
- US 5597936 A
- US 5581005 A
- US 5559261 A
- US 5169552 A
- US 4714562 A
- EP 0242325 A
- US 4518615 A
- US 5093136 A
- US 5266336 A
- US 5601858 A
- US 6986709 B
- US 6162423 A
- US 5968286 A
- US 5935561 A
- US 5932203 A
- US 5837661 A
- US 5776443 A
- US 5756436 A
- US 5661118 A
- US 5618523 A
- US 5275755 A
- US 5085857 A
- US 4673568 A
- US 4387090 A
- US 4705681 A
- US 8299011 B

**Non-patent literature cited in the description**

- **LEE et al.** *J. Microencapsulation,* 2002, vol. 19, 559-569